(19) Europäisches Patentamt
European Patent Office
Office européen des brevets

(11) **EP 1 717 574 B1**

(12) **EUROPÄISCHE PATENTSCHRIFT**

(45) Veröffentlichungstag und Bekanntmachung des Hinweises auf die Patenterteilung:
**06.11.2019 Patentblatt 2019/45**

(51) Int Cl.:
***G01N 33/487*** *(2006.01)*

(21) Anmeldenummer: **06008658.4**

(22) Anmeldetag: **26.04.2006**

(54) **Detektion der Abdichtung einer biologischen Substanz auf einem Träger mittels Rauschanalyse**

Detection of sealing of biological material on a substrate by means of noise analysis

Détection du joint de matière biologique sur un support à l'aide d'une analyse de bruit

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **26.04.2005 DE 102005019191**

(43) Veröffentlichungstag der Anmeldung:
**02.11.2006 Patentblatt 2006/44**

(73) Patentinhaber: **Venneos GmbH**
**70569 Stuttgart (DE)**

(72) Erfinder: **Völker, Moritz**
**80805 München (DE)**

(74) Vertreter: **Müller Hoffmann & Partner**
**Patentanwälte mbB**
**St.-Martin-Strasse 58**
**81541 München (DE)**

(56) Entgegenhaltungen:
**US-A1- 2003 113 833    US-B1- 6 611 151**

- YANG M ET AL: "Influence of geometry and environmental parameters on the quality of signature patterns for single neuron chemical sensors" SENSORS AND ACTUATORS B, ELSEVIER SEQUOIA S.A., LAUSANNE, CH, Bd. 104, Nr. 1, 3. Januar 2005 (2005-01-03), Seiten 163-171, XP004645561 ISSN: 0925-4005
- GIAEVER I ET AL.: "A morphological biosensor for mammalian cells" NATURE, Bd. 366, 9. Dezember 1993 (1993-12-09), Seiten 591-592, XP002393348
- WEGENER J ET AL.: "Barrier function of porcine choroid plexus epithelial cells is modulated by cAMP-dependent pathways in vitro" BRAIN RESEARCH, Bd. 853, 2000, Seiten 115-124, XP002393349
- BUITENWEG J R ET AL: "MEASUREMENT OF SEALING RESISTANCE OF CELL-ELECTRODE INTERFACES IN NEURONAL CULTURES USING IMPEDANCE SPECTROSCOPY" MEDICAL AND BIOLOGICAL ENGINEERING AND COMPUTING, PETER PEREGRINUS LTD. / IFMBE, HERTS, GB, Bd. 36, Nr. 5, 1. September 1998 (1998-09-01), Seiten 630-637, XP000777559 ISSN: 0140-0118

EP 1 717 574 B1

**Beschreibung**

Die Erfindung betrifft ein Verfahren zur Bestimmung der Abdichtung einer Substanz auf einem Träger durch Bestimmen des elektrischen Rauschens.

Der Vorgang der selbständigen bzw. aktiv vorgenommenen Anlagerung einer Substanz auf einen Träger kann zahlreiche Informationen über die Substanz, den Träger und deren Interaktion liefern: Bei einer aktiv vorgenommenen Anlagerung, z.B. bei mechanischem Anpressen eines Objektes auf einen Träger, hängt die Innigkeit des Kontakts vom Anpressdruck, von der Elastizität des Objekts und von der Rauigkeit von Objekt und Träger ab. Bei einer selbständigen Anlagerung, z.B. einer Lipidmembran auf einen Träger, hängt die Abdichtung von der Zusammensetzung der Lipidmembran und von den chemischen Oberflächeneigenschaften des Trägers ab.

Ein konkretes Beispiel der selbständigen Anlagerung ist die Zelladhäsion. Zelladhäsion bezeichnet das Aneinanderhaften von biologischen Zellen untereinander und von Zellen an Substraten. Sie spielt eine ganz zentrale Rolle in der Entwicklung zum erwachsenen Organismus. In allen mehrzelligen Lebewesen wird bei der Entwicklung zum erwachsenen Organismus und bei der Ausbildung von Organen die Zelladhäsion ständig geregelt, um den Zusammenhalt von Zellverbänden zu stärken oder zu vermindern. Außerdem kontrolliert sie die Funktion aller Epithel- und Endothelstrukturen, z.B. des Darms, der Blase, der Blutgefäße und der Blut-Hirn-Schranke.

Die Bedeutung der Zelladhäsion sei an zwei konkreten Beispielen ausgeführt: Epithele und Metastasen von Krebszellen.

Epithele bestehen aus einer ein- oder mehrlagigen Schicht von Epithelzellen. Sie kleiden alle inneren Hohlräume von Lebewesen aus und dienen der Abgrenzung des Körpers von der Umwelt. Der wichtigste Faktor ist die Durchlässigkeit der Epithele für Wasser, Ionen, Proteine und sogar für ganze Zellen. Gesteuert wird die Durchlässigkeit von dem lateralen Zusammenhalt der Epithelzellen untereinander. Das Ausbilden so genannter "tight junctions" zwischen den Zellen verhindert, dass die entsprechenden Stoffe von der körperfremden (apikalen) Seite des Epithels auf die körpereigene (basale) Seite diffundieren oder umgekehrt. Die gewünschten Konzentrationsunterschiede werden durch Regulation der tight junctions sowie durch aktive Transportprozesse durch die Epithelzellen eingestellt.

In der Krebsforschung interessiert die Zelladhäsion, da die Gefährlichkeit eines Krebstumors in erster Linie von seiner Fähigkeit zur Metastasenbildung abhängt. Gutartige Tumore wachsen zwar, doch solange der Zellverband geschlossen bleibt, kann der Tumor operativ meist vollständig entfernt werden. Bei bösartigen Tumoren können sich einzelne Zellen oder kleine Zellverbände aufgrund von Veränderungen in der Zelladhäsion vom Haupttumor lösen und im Körper verteilen, was eine operative Entfernung kaum noch möglich macht. Ein Verfahren und eine Vorrichtung zur Untersuchung der Zelladhäsion ist daher für die Forschung an den Mechanismen der Tumorentwicklung interessant sowie von Bedeutung für die Entwicklung von Krebspharmaka, die eine Zellablösung aus dem Tumor oder eine Anheftung von Krebszellen an anderen Stellen im Körper verhindern und so die Gefährlichkeit von Tumoren senken könnten.

Im Stand der Technik sind Verfahren bekannt, in denen die Zelladhäsion durch Messung der Impedanz durch Anwenden eines Wechselstroms oder/und einer Wechselspannung bestimmt wird.

Unter einer Impedanz $Z$ versteht man den frequenzabhängigen Widerstand eines elektrisch leitfähigen Materials. Die (komplexwertige) Impedanz setzt sich aus dem Wirkwiderstand und Blindwiderstand zusammen. Der Wirkwiderstand entspricht dem Realteil $Re(Z)$. Der Wirkwiderstand ist nicht frequenzabhängig. Der Blindwiderstand entspricht dem frequenzabhängigen Imaginäranteil von $Z$, der entweder ein kapazitiver oder induktiver Blindwiderstand sein kann. Im Folgenden wird der kapazitive Blindwiderstand auch einfach kapazitiver Widerstand genannt. Für die vorliegende Erfindung hat der induktive Blindwiderstand nur eine geringe Bedeutung.

Wenn die Fläche einer Elektrode verkleinert wird (bei sonst unveränderten Oberflächeneigenschaften), steigt die Impedanz der Elektrode an. Der Wirkwiderstand ist umgekehrt proportional zur Elektrodenfläche und steigt bei Verkleinerung der Elektrodenfläche entsprechend an. Der kapazitive Blindwiderstand ist umgekehrt proportional zur Elektrodenkapazität und steigt bei Verkleinerung der Elektrodenfläche an, da sich durch die Verkleinerung der Fläche die Kapazität anteilig vermindert. Bei Elektroden, die in Kontakt mit einer elektrisch leitfähigen Flüssigkeit sind, kann der Wirkwiderstand im allgemeinen vernachlässigt werden. Es dominiert der kapazitive Blindwiderstand.

Das existierende Verfahren, um über elektrische Messungen die Zelladhäsion zu charakterisieren, ist das Electrode-Cell-Impedance-Sensing (ECIS, Giaever und Keese, Nature 366:591-592, 1993). Dabei werden Zellen auf Metallelektroden kultiviert. Durch Anlegen einer Spannung an die Elektrode (gegenüber einer Referenzelektrode im Bad) und durch Messung des fließenden Stroms wird die Impedanz des Systems Elektrode-Zelle-Bad bestimmt. Die Veränderung dieser Impedanz während der Kultur (typischerweise mehrere Stunden) lässt Rückschlüsse auf das Wachstum, die Adhäsion, das Ausbreitungsverhalten, die Beweglichkeit und auf die Barrierenwirkung der kultivierten Zellen zu.

Im ECIS-Verfahren führt der Stromfluss über die Grenze Elektrode-Elektrolyt zu elektrochemischen Prozessen, die nicht genau bekannt sind. Die Auswirkungen dieser elektrochemischen Prozesse auf die Messergebnisse lassen sich demnach nur schwer bestimmen.

Aufgrund des Stromflusses über die Grenze Elektrode-Elektrolyt wirkt sich im ECIS-Verfahren die Impedanz der Elektrode mit ihrem Real- und Imaginäranteil auf das Messergebnis aus. Wegen des Anstiegs der Impedanz bei Verkleinerung der Elektrode können im ECIS-Verfahren nur Elektroden mit einer bestimmten Mindestgröße eingesetzt werden, die nicht unterschritten werden kann. In der Praxis werden im ECIS-Verfahren Elektroden verwendet, die einen Durchmesser von mindestens 100 $\mu$m bis 200 $\mu$m haben. Diese Größe liegt oberhalb der Größe einer einzelnen Zelle in Kultur, die auf einem Substrat adhäriert. Wegen der notwendigen Mindestelektrodengröße erlaubt das ECIS-Verfahren nicht, die Adhäsion einer einzelnen Zelle auf einem Substrat zu messen. Mit dem ECIS-Verfahren können z.B. Schichten von Epithelzellen untersucht werden.

Im Stand der Technik wird die Impedanz ferner ausgenutzt, um Niederschläge auf Elektroden zu bestimmen. US 4,920,047 beschreibt ein Verfahren zur Bestimmung einer immunologisch aktiven Substanz durch die Änderung der Impedanz einer Elektrode. Eine Elektrode wird so vorbehandelt, dass sie entweder die immunologisch aktive Substanz bindet oder aber ein Enzym. Wenn keine immunologisch aktive Substanz in der Probe vorhanden ist, kann das gebundene Enzym aus geeigneten Substraten einen unlöslichen Niederschlag auf der Elektrode bilden. Dieser Niederschlag verändert die Impedanz der Elektrode, was die Bestimmung der Konzentration der immunologisch aktiven Substanz erlaubt. US 4,920,047 bestimmt die Impedanz durch Anwenden eines Wechselstroms. Aufgrund des über die Grenzfläche Elektrode-Elektrolyt fließenden Stromes hat das in US 4,920,047 beschriebene Verfahren dieselben Nachteile wie das oben beschriebene Verfahren ECIS-Verfahren (Grenzflächenprozesse zwischen Elektrode und Elektrolyt, stärkerer Einfluss der Impedanz auf das Messergebnis bei kleineren Elektroden).

Es sind aus dem Stand der Technik Verfahren bekannt, die Spannungsrauschen und Stromrauschen als Messgrößen zur Bestimmung des Korrosionszustandes von Metalloberflächen einsetzen. Durch die Korrosion finden Passivierungs/Aktivierungsvorgänge auf der Metalloberfläche statt, die zu Schwankungen der Ladung an der Grenzfläche zwischen Metall und Elektrolyt führen. Die zu untersuchende Metalloberfläche verändert sich also stetig. Die Verfahren sind auf die Messung des Strom- bzw. Spannungsrauschens an der zu untersuchenden Metalloberfläche ausgelegt, wobei die Oberfläche als Elektrode eingesetzt wird. Der Elektrolyt bleibt während der Messung unverändert.

Bei Korrosionsuntersuchungen werden die Begriffe "Elektrochemisches Rauschen (electrochemical noise, ECN)" oder "Elektrochemische Rauschanalyse" verwendet. US 6,611,151 beschreibt ein Verfahren zur Untersuchung beschichteter metallischer Oberflächen anhand des elektrochemischen Rauschens. US 6,611,151 verwendet hierzu eine Anordnung aus drei Elektroden in einem Elektrolyt. Die erste Elektrode ist das zu untersuchende Material, die zweite Elektrode ist eine Referenzelektrode (z.B. Ag/AgCl), und die dritte Elektrode ist eine "witness"-Elektrode aus einem edleren Metall (z.B. Platin). Zwischen der Referenzelektrode und dem zu untersuchenden Material wird das Spannungsrauschen gemessen, und zwischen der "witness"-Elektrode und dem Material wird gleichzeitig das Stromrauschen bestimmt.

In US 5,888,374 wird ein Verfahren zur Bestimmung von Korrosionsvorgängen durch Lochfraß offenbart. Hierzu wird das elektrochemische Rauschen des zu untersuchenden Materials (z.B. korrodierende Rohrleitungen) in einer Anordnung aus drei Elektroden und Elektrolyt zur Messung des Strom- und Spannungsrauschens bestimmt, wobei das zu untersuchende Material eine dieser Elektroden darstellt. Der Lochfraß bewirkt im Bereich sehr niedriger Frequenzen ($10^{-4}$-$10^{-5}$ Hz) eine Abnahme der spektralen Leistungsdichte mit ansteigender Frequenz. Das Rauschen einer gleichmäßigen Korrosion ist von der Frequenz nahezu unabhängig.

Vasilescu et al. (Electrochimica Acta, 1974, 19:181-186) beschreibt die Rauschleitfähigkeit ("noise conductivity") von Elektrolyten, die mittels Metallelektroden aus dem thermischen Rauschen ohne Einsatz einer externen Spannung bestimmt wurde. Das durch das Elektrolyt erzeugte Rauschen entspricht gemäß Vasilescu et al. (supra) einem weißen Rauschen. Es ist also von der Frequenz unabhängig. Dieser Ansatz wurde jedoch später nicht aufgegriffen, um Verfahren zur verbesserten Impedanzmessung zu entwickeln.

M. Yang et al., "Influence of geometry and environmental parameters on the quality of signature patterns for single neuron chemical sensors", SENSORS AND ACTUATORS B104 (2005-01-03) 163-171 beschreibt den Einfluß der Geometrie und von Umgebungsparametern auf die Qualität der Signalübertragung von Einzelneuronen auf Elektroden. Hierbei wird festgestellt, dass bei einer hohen Abdichtimpedanz das Signal/Rausch-Verhältnis gut ist.

US 2003/113833 A1 beschreibt eine Vorrichtung zur Messung extrazellulärer Signale von Zellen auf Elektroden. Hierbei wird die Zelle durch ein Durchgangsloch in einem Chip angesaugt und ein Strom durch Ionenkanäle gemessen.

J. Wegener et al., "Barrier function of porcine choroid plexus epithelial cells is modulated by cAMP-dependent pathways in vitro", rain Research 853 (2000) 115-124 beschreibt eine Impedanzanalyse von Epithelzellen, die auf einem Trägersubstrat mit Elektroden angeordnet sind.

Giaever I. et al.: "A morphological biosensor for mammalian cells", Nature, Vol. 366, 9. Dezember 1993 befasst sich mit einem sogenannten Electrode-Cell-Impedance-Sensing (ECIS) System. Dabei werden Zellen auf Metallelektroden kultiviert. Durch Anlegen einer Spannung an die Elektrode und durch Messung des fließenden Stroms wird die Impedanz des Systems Elektrode-Zelle-Bad bestimmt. Hierbei werden, nachdem die Zellen sich komplett über der Elektrodenfläche ausgebreitet haben, die Fluktuationen der gemessenen Impedanz erfasst. Diese Fluktuationen sind das Ergebnis einer konstanten Bewegung der Zellen, welche den Stromfluss in geringfügiger Weise verändern. Sowohl die endgültige Durchschnittsimpedanz als auch die Fluktuationen sind charakteristisch für den Zelltyp und können als Zellsignatur oder Fingerabdruck verwendet werden.

Buitenweg J.R. et al.: "Measurement of sealing resistance of cell-electrode interfaces in neuronal cultures using impedance spectroscopy", Medical and Biological Engineering and Computing, Bd. 36, Nr. 5, 1. September 1998, Seiten 630 - 637, XP 000777559 befasst sich mit der Messung eines Abdichtwiderstands einer Zell-Elektroden-Schnittfläche in neuronalen Kulturen unter Verwendung von Impedanzspektroskopie. Bei dieser Methode wird eine Wechselspannung zwischen Referenzelektrode und Messelektrode angelegt und die frequenzabhängige Übertragungsfunktion von dem elektrischen Badpotential in den Spaltbereich zwischen auf der Elektrode anhaftender Zelle und Elektrode bestimmt.

Die Aufgabe der vorliegenden Erfindung ist die Bereitstellung eines Verfahrens zur Bestimmung der Abdichtung einer Substanz auf einem Träger, das die oben beschriebenen Nachteile der Bestimmung der Abdichtung durch Impedanzmessung mittels Anwenden von Spannungen oder/und Strömen ganz oder teilweise überwindet. Durch die vorliegende Erfindung soll der Nachteil bestehender Verfahren überwunden werden, dass die verwendeten Elektroden eine bestimmte Mindestgröße haben müssen, damit die Elektrodenimpedanz die Messung nicht stört.

Insbesondere ist es die Aufgabe der Erfindung, ein verbessertes Verfahren zur Bestimmung der Adhäsion einer Substanz auf einem Träger bereitzustellen, z.B. einer biologischen Substanz wie z.B. einer Zelle.

Keines der Verfahren aus dem Stand der Technik nutzt das Rauschen des Elektrolyts als Messgröße zur Bestimmung der Abdichtung oder/und Adhäsion einer Substanz, insbesondere einer biologischen Substanz, auf einem Träger. Eine Lösung ist daher ein Verfahren zur Bestimmung der Abdichtimpedanz eines mit einer elektrisch leitfähigen Flüssigkeit gefüllten Spaltes zwischen einer Substanz und einem Träger gemäß Ansprüche 1-23.

In einer Ausführungsform umfasst das Verfahren weiterhin den Schritt

(d) Bestimmen der Adhäsion der Substanz auf dem Träger aus der Abdichtung der Substanz auf dem Träger.

Unter dem Rauschen einer Größe versteht man deren zeitliche Fluktuationen. Die Fluktuationen sind dadurch gekennzeichnet, dass sie sich nichtdeterministisch verhalten, d.h. eine zeitliche Vorhersage des Signals ist nicht möglich. Die Fluktuationen sind spontanen Ursprungs, stammen also nicht von einer externen Quelle, sondern rühren vom System selbst her und lassen sich nicht vermeiden oder unterdrücken.

Jede elektrische Impedanz rauscht. Exakt formuliert: An den Enden einer elektrischen Impedanz lässt sich eine zeitlich fluktuierende Spannung unabhängig von äußeren Spannungen und Feldern messen, die im Kontext der vorliegenden Erfindung als Rauschspannung $V(t)$ bezeichnet wird. Das erfindungsgemäße Verfahren beruht darauf, dass das Rauschen zwischen zwei Elektroden, die beide in Kontakt mit einer elektrisch leitfähigen Flüssigkeit stehen, stärker wird, wenn die Impedanz der elektrisch leitfähigen Flüssigkeit ansteigt. In Anbetracht der Tatsache, dass ein Anstieg des Rauschens mit einer größeren Abdichtung korreliert, kann die Messung der Impedanz einer elektrisch leitfähigen Flüssigkeit zur Bestimmung der Abdichtung einer Substanz auf einem Träger verwendet werden.

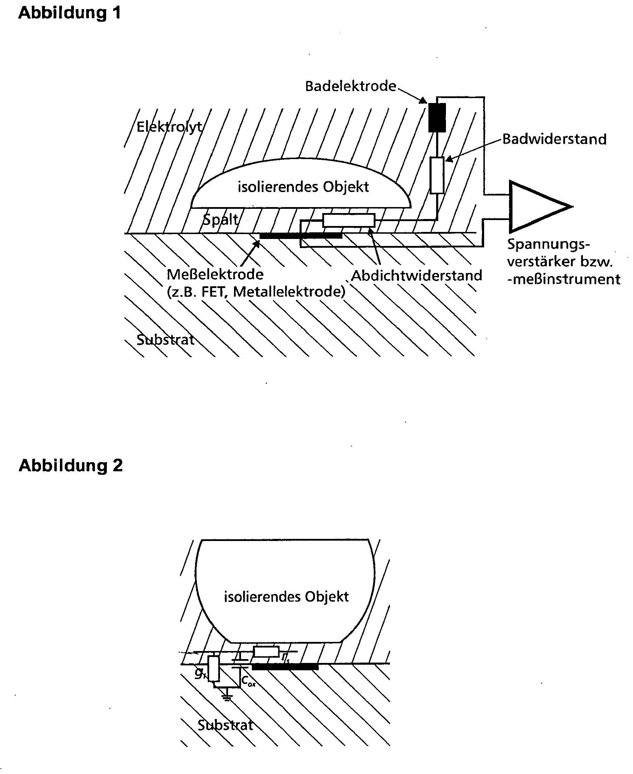

Abbildung 1 zeigt den schematischen Aufbau einer Vorrichtung, die zur Messung des Spannungsrauschens im erfindungsgemäßen Verfahren geeignet ist. Eine spannungssensitive Elektrode (Messelektrode), umfasst von einem Träger, ist in Kontakt mit der darüber liegenden elektrisch leitfähigen Flüssigkeit (Elektrolyt), in der sich eine zweite Elektrode befindet, die als Bezugselektrode verwendet wird.

Durch aktive oder selbständige Anlagerung einer elektrisch isolierenden (oder hinreichend schlecht leitenden) Substanz entsteht zwischen Elektrode und Substanz ein Spalt mit einer elektrischen Impedanz, der Abdichtimpedanz. Das erfindungsgemäße Beispiel 1 zeigt, dass eine auf die Elektrodenfläche gedrückte Silikonperle zu einem deutlichen Anstieg der spontanen zeitlichen Fluktuationen (d.h. des Rauschens) zwischen der mindestens einen ersten Elektrode und der zweiten Elektrode führt. Die Silikonperle dichtet hierbei die gesamte Elektrodenfläche gegenüber der umgebenden elektrisch leitfähigen Flüssigkeit ab (siehe Abbildung 2), was eine Erhöhung der Impedanz zwischen den beiden Elektroden zur Folge hat. Das erfindungsgemäße Beispiel 2 demonstriert den Anstieg des Rauschens, das an Elektroden gemessen wird, die durch eine lebende Zelle gegenüber der umgebenden elektrisch leitfähigen Flüssigkeit abgedichtet werden.

Im erfindungsgemäßen Verfahren kann das Rauschen genutzt werden, um daraus eine andere Größe zu ermitteln: den reellen Teil der im allgemeinen komplexwertigen Impedanz zwischen den Elektroden. Die so ermittelte Impedanz kann entweder bereits die gewünschte Größe sein oder anhand eines geeigneten Modells der Abdichtung zur Bestimmung anderer Parameter, z.B. der Breite des Spalts, verwendet werden.

Somit kann im erfindungsgemäßen Verfahren das elektrische Rauschen der Impedanz zwischen der mindestens einen ersten Elektrode umfasst von einem Träger und einer zweiten Elektrode zur Bestimmung der Abdichtung der Substanz herangezogen werden, sofern die Substanz die mindestens eine erste Elektrode ganz oder teilweise bedeckt.

Im Sinne der vorliegenden Erfindung beschreibt "Abdichtung einer Substanz auf dem Träger" allgemein den mit elektrisch leitfähiger Flüssigkeit gefüllten Spalt zwischen der vom Träger umfassten Elektrode und der Substanz. Der "Spalt" umfasst den Raum zwischen Substanz und Träger, und außerdem alle Strukturen zwischen Substanz und Träger und auch Strukturen innerhalb der Substanz, die einen Beitrag zur Abdichtung der Substanz auf dem Träger leisten können. Zum Beispiel kann die Abdichtung einer lebenden Zelle auf einem Träger durch Einlagerung von Substanzen in den Raum zwischen Zelle und Träger ansteigen. Ein weiteres Beispiel für Strukturen, die zur Abdichtung beitragen, aber nicht zum geometrischen Raum zwischen Substanz und Träger gehören, sind tight junctions, die lebende Zellen miteinander verbinden.

Je kleiner bzw. dichter der Spalt ist, desto größer ist der Kontakt zwischen Substanz und Träger und desto größer ist die Abdichtung der Substanz auf dem Träger. Je größer die Abdichtung ist, desto größer ist folglich die Impedanz des Spalts. Die Impedanz des Spalts wird im Folgenden daher auch als Abdichtimpedanz bezeichnet. Ohne Substanz auf dem Träger existiert kein Spalt und auch keine Abdichtimpedanz. Anhand des Rauschens kann die Abdichtung bestimmt werden, wobei die Abdichtung umso größer ist, je stärker das Rauschen ist.

Typischerweise beträgt die Abdichtimpedanz bei der Adhäsion einzelner Zellen in normalem Kulturmedium 500 k$\Omega$ bis 10 M$\Omega$, bei dichten Zellschichten wie Epithelzellen 500 k$\Omega$ bis 50 M$\Omega$, bei aktiv angedrückten Materialien (wie z.B. die Silikonperle in Beispiel 1) 1 M$\Omega$ bis 1 G$\Omega$.

Je nachdem, welche Parameter der zu untersuchenden Substanz, z. B. einer Zelle, eines Zellverbandes oder einer Zellmembran, bekannt sind, lassen sich aus der Abdichtung bzw. Abdichtimpedanz auf dem Träger Rückschlüsse über den Zustand oder/und die Veränderung der Substanz auf dem Träger ziehen.

Die Rauschspannung V(t) einer (im allgemeinen komplexwertigen) Impedanz Z hat die spektrale Leistungsdichte

$$S_V(f) = 4k_B T \cdot \mathrm{Re}(Z) \qquad\qquad\qquad (I),$$

wobei $k_B$ die Boltzmannkonstante, $T$ die absolute Temperatur und $\mathrm{Re}(Z)$ der Realteil der Impedanz ist. Diese Beziehung gilt ganz allgemein für elektrische Impedanzen, unabhängig vom Transportmechanismus, von der Art der Ladungsträger und von äußeren Spannungen und Feldern. Sie ist im thermodynamischen Gleichgewicht streng gültig und auch für viele Ungleichgewichtszustände, insbesondere stromdurchflossene Impedanzen, eine gute Näherung. Man kann die Ursache des Spannungsrauschens in der Brownschen Molekularbewegung der geladenen Teilchen (Ionen im Elektrolyten, Elektronen in Metallen) oder als Folge thermodynamischer Grundgleichungen ansehen. Beide Betrachtungsweisen sind gleichwertig.

Im erfindungsgemäßen Verfahren setzt sich die Gesamtimpedanz zwischen der ersten und der zweiten Elektrode aus vier Teilen zusammen: Der Impedanz der ersten Elektrode, der Impedanz der zweiten Elektrode, der Impedanz der elektrisch leitfähigen Flüssigkeit und der Abdichtimpedanz.

Sowohl die Abdichtimpedanz als auch die Elektrodenimpedanzen sind komplexwertig (sowohl Real- als auch Imaginärteil ungleich Null), wobei bei der Abdichtimpedanz der Realteil dominiert, bei den Elektroden der Imaginärteil.

In Schritt (a) des erfindungsgemäßen Verfahrens wird die mindestens eine erste Elektrode ganz oder teilweise von der Substanz, deren Abdichtung oder/und Adhäsion bestimmt werden soll, bedeckt. In einer bevorzugten Ausführungsform ist die mindestens eine erste Elektrode zu mindestens 70 %, stärker bevorzugt mindestens 90 %, noch stärker bevorzugt mindestens 95 %, am stärksten bevorzugt ganz durch die Substanz bedeckt. Sofern mehr als eine erste Elektrode eingesetzt wird, können die Elektroden unabhängig voneinander ganz oder teilweise mit der Substanz bedeckt sein.

In Schritt (b) des erfindungsgemäßen Verfahrens wird das elektrische Rauschen zwischen zwei Elektroden bestimmt. Es ist bevorzugt, dass die Bestimmung des Rauschens in Schritt (b) im wesentlichen ohne Anlegen einer Spannung und eines Stroms zwischen der mindestens einen ersten und der zweiten Elektrode erfolgt (im wesentlichen spannungs- und stromfreie Messung). Das Rauschen kann somit nahe am thermodynamischen Gleichgewicht bestimmt werden. Nahe dem thermodynamischen Gleichgewicht wird das Rauschen von der Impedanz zwischen der mindestens einen ersten Elektrode und der zweiten Elektrode in der elektrisch leitfähigen Flüssigkeit (Elektrolyt) bestimmt. Diese Ausführungsform hat den Vorteil, dass Vorrichtungen zur Spannungs- und Stromanwendung, z.B. zur Modulation einer Elektrodenspannung oder/und eines Elektrodenstromes, wie sie in Verfahren des Standes der Technik zur Impedanzmessung benötigt werden, entfallen können.

Unter einer im wesentlichen spannungs- und stromfreien Messung wird erfindungsgemäß eine Messung verstanden, bei der sich die mindestens eine erste Elektrode und die zweite Elektrode jeweils weitgehend auf demselben Potential wie die umgebende elektrisch leitfähige Flüssigkeit befinden (spannungsloser Zustand). Damit fließt im wesentlichen kein Strom über die Grenzflächen der Elektroden zu der elektrisch leitfähigen Flüssigkeit (stromfreier Zustand). Die im wesentlichen spannungs- und stromfreie Messung hat den Vorteil, dass elektrochemische Reaktionen an der Grenzfläche Elektrode-Elektrolyt wegen fehlendem Stromfluss nicht auftreten. Damit entfällt im erfindungsgemäßen Verfahren elektrochemisches Rauschen als mögliche Quelle von Störungen der Messungen.

Das elektrische Rauschen einer Impedanz kann als Strom- und als Spannungsrauschen bestimmt werden. In einer weiteren bevorzugten Ausführungsform der Erfindung wird das elektrische Rauschen der Impedanz in Schritt (b) als Rauschspannung V(t) bestimmt.

Die typische Amplitude des Rauschens lässt sich direkt aus dem Widerstandswert ermitteln. Das Spannungsrauschen eines Widerstands R zeigt im Frequenzbereich $f_1$ bis $f_2$ einen RMS-Wert von

$$V_{rms} = \sqrt{\int_{f_1}^{f_2} 4k_B T \cdot R \cdot df} = \sqrt{4k_B T \cdot R \cdot \left(f_2 - f_1\right)} \qquad \text{(II)}.$$

Anstelle von $R$ kann auch der Wert $Re(Z)$ einer Impedanz $Z$ in die Formel eingesetzt werden.

Damit ergibt sich, dass die spektrale Leistungsdichte und der RMS-Wert nur vom Wirkwiderstand $Re(Z)$ einer Impedanz $Z$ abhängen.

Zwar ist ein Peak-Peak-Wert für eine rauschbehaftete Größe nicht wohldefiniert, aber wenn man eine ungefähre Peak-Peak-Amplitude benötigt, kann man die Beziehung

$$V_{peak-peak} \approx 6{,}6 \cdot V_{rms} \qquad \text{(III)}$$

benutzen. Der Faktor 6,6 entstammt dabei der Annahme, dass man als Peak-Peak-Bereich denjenigen Bereich ansieht, in dem sich das Signal 99,9 % der Zeit bewegt. Dieser Bereich entspricht bei einer Gaußverteilung etwa $\pm 3{,}2\sigma$.

In einer weiteren bevorzugten Ausführungsform des erfindungsgemäßen, Verfahrens wird die Abdichtung einer Substanz bestimmt, indem in Schritt (c) die Stärke des Rauschens bestimmt wird. Stärkeres (schwächeres) Rauschen geht z.B. mit einer Erhöhung (Verringerung) der Amplitude einher. In dieser Ausführungsform kann die Zunahme bzw. Abnahme des gemessenen Rauschens relativ zu dem Rauschen bei einer vorgegebenen Abdichtung bestimmt werden. Damit kann direkt die stärkere bzw. schwächere Abdichtung der Substanz in Bezug auf die vorgegebene Abdichtung bestimmt werden. Hierzu kann z B. eine Schwelle definiert werden, die über- bzw. unterschritten werden muss. Es ist bevorzugt, dass die Stärke des Rauschen in einem vorbestimmten Frequenzbereich $f_1$ bis $f_2$ bestimmt wird.

In einer anderen bevorzugten Ausführungsform des erfindungsgemäßen Verfahrens wird in Schritt (c) die Abdichtung der Substanz auf dem Träger aus der Peak-Peak-Amplitude der Rauschspannung in einem vorbestimmten Frequenzbereich $f_1$ bis $f_2$ bestimmt. Hierbei ist es bevorzugt, dass die Peak-Peak-Amplitude nach

$$V_{peak-peak} \approx 6{,}6 \cdot V_{rms} \qquad \text{(III)}$$

berechnet wird. Wegen der Proportionalität kann ein beliebiger anderer Faktor als 6,6 verwendet werden. Insbesondere kann auch direkt der $V_{rms}$-Wert als Maß für die Peak-Peak-Amplitude verwendet werden.

In noch einer anderen bevorzugten Ausführungsform des erfindungsgemäßen Verfahrens wird in Schritt (c) die Abdichtung der Substanz auf dem Träger aus der Amplitude der Rauschspannung in einem vorbestimmten Frequenzbereich $f_1$ bis $f_2$ bestimmt, wobei die Amplitude durch den Spannungsbereich gekennzeichnet ist, in dem sich die elektrische Rauschspannung für bis zu 95 %, vorzugsweise bis zu 99 %, stärker bevorzugt bis zu 99,9 % eines vorbestimmten Zeitraums bewegt.

In einer weiteren bevorzugten Ausführungsform des erfindungsgemäßen Verfahrens wird in Schritt (c) die Abdichtung der Substanz auf dem Träger aus der Amplitude der Rauschspannung in einem vorbestimmten Frequenzbereich $f_1$ bis $f_2$ bestimmt, wobei die Amplitude der Rauschspannung durch $\pm 3{,}2\sigma$ gekennzeichnet ist. $\sigma$ ist die Standardabweichung der Verteilung der einzelnen über eine vorbestimmte Zeit gemessenen Werte der Rauschspannung. Es kann ebenso ein beliebiger anderer Faktor als 3,2 oder/und $\sigma^2$ anstatt von $\sigma$ verwendet werden. Insbesondere kann auch direkt $\sigma$ als Maß für die Amplitude verwendet werden.

Der Fachmann kann die elektrischen Eigenschaften von Substanzen auf Trägern durch Ersatzschaltbilder sehr gut annähern, die die beteiligten Komponenten mit passiven und aktiven elektrischen Bauelementen beschreiben. Passive elektrische Bauelemente können ohmsche Widerstände/Leitfähigkeiten oder/und Kapazitäten sein. Aktive elektrische Bauelemente können Spannungsquellen (z.B. Nernst-Potentiale über Membranen) oder/und Gleichrichter (z.B. Ionenkanäle) sein.

Der Fachmann kann Ersatzschaltbilder gleichermaßen für nichtbiologische und biologische Substanzen erstellen, die gemäß Schritt (a) der erfindungsgemäßen Verfahren auf einem Träger bereitgestellt werden.

Eine zusammenfassende Darstellung der Ersatzschaltbilder lebender Zellen findet sich z B. in Hille, B., "Ionic Channels of Excitable Membranes", Sinauer Associates Inc., Sunderland, Massachusetts, 1991.

Mithilfe eines Ersatzschaltbildes der Anordnung aus der mindestens einen ersten Elektrode, der zweiten Elektrode in der elektrisch leitfähigen Flüssigkeit und der Substanz kann der Fachmann aus der Abdichtimpedanz die Kenngrößen bestimmen, die ein Maß für die Adhäsion der Substanz auf dem Träger sind. Ein Maß für die Adhäsion kann der Widerstand $r_s$ des Raumes zwischen Substanz und dem Träger sein oder die Abdichtimpedanz, die durch die Dichtigkeit der Substanz, die Breite des Spalts zwischen der Zelle und der Substanz oder/und die Schichtpermeabilität (z.B. die Impedanz von tight junctions) definiert wird (siehe oben).

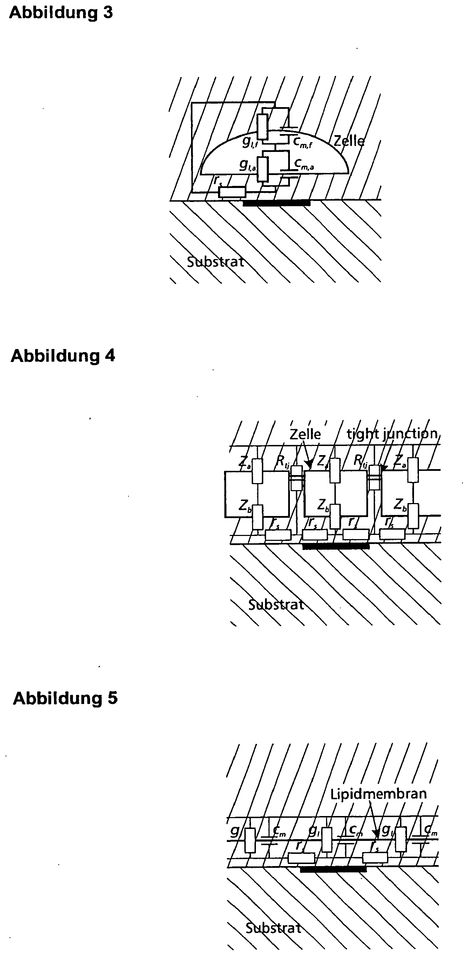

Die folgenden drei Beispiele beziehen sich auf biologische Substanzen, die zu charakteristischen Abdichtimpedanzen führen. Da das erfindungsgemäße Verfahren vorzugsweise strom- und spannungsfrei arbeitet (siehe oben), sind in den Ersatzschaltbildern der Abbildungen 3 bis 5 aus Gründen der Vereinfachung lediglich die passiven Bauelemente gezeigt.

Bei einer einzelnen Zelle auf einem Sensor (Abb. 3) wird die Abdichtimpedanz aus dem Widerstand $r_s$, den Leckleitfähigkeiten $g_{l,f}$ und $g_{l,a}$ und den Membrankapazitäten $c_{m,f}$ und $c_{m,a}$, jeweils für die freie bzw. die adhärierte Zellmembran gebildet. Die Leckleitfähigkeiten sowie die Membrankapazitäten sind meist recht genau bekannt, so dass der Widerstand $r_s$ durch dem Fachmann bekannte Verfahren zur Schaltungsberechnung mithilfe der bekannten Parameter aus der Abdichtimpedanz bestimmt werden kann. Diese ist maßgeblich von der Breite des Spalts zwischen Zelle und Träger bestimmt und gibt so Auskunft über die Zell-Träger-Adhäsion und über deren Änderung z.B. bei Zugabe von Wirkstoffen.

Bei einer Schicht aus Ephitelzellen (Abb. 4) bilden sich zwischen den Zellen so genannte "tight junctions" aus, die als Diffusionsbarriere für Wasser und Ionen dienen und damit eine hohe Impedanz aufweisen. Einzelne Epithelzellen der Schicht können wie einzelne Zellen aus Abb. 3 oder durch die Impedanzen der freien ($Z_a$) bzw. der adhärierten Zellmembran ($Z_b$) beschrieben werden. Der Widerstand $R_{tj}$ der tight junctions ist einer der wichtigsten Parameter einer Ephitelstruktur und gibt daher in direkter Weise Auskunft über deren Funktionieren. Anhand der gegebenen Parameter kann der Fachmann den Widerstand der tight junctions $R_{tj}$ oder deren Impedanz aus der Abdichtimpedanz durch bekannte Verfahren zur Schaltungsberechnung bestimmen. Eine Veränderung der tight junctions, z.B. bei Zugabe eines Wirkstoffs, kann als Test für die Wirksamkeit des Wirkstoffs verwendet werden.

Bei einer Lipiddoppelmembran (Abb. 5), auch als "supported lipid bilayer" bezeichnet, wird die Abdichtimpedanz aus dem Widerstand $r_s$ sowie aus der Kapazität $c_m$ und der Leckleitfähigkeit $g_l$ der Lipidmembran gebildet. Anhand der gegebenen Parameter kann der Fachmann den Widerstand $r_s$ oder die entsprechende Impedanz aus der Abdichtimpedanz durch bekannte Verfahren zur Schaltungsberechnung bestimmen. Eine Lipidmembran stellt ein einfaches Modellsystem für die Membranen biologischer Zellen dar. So kann man Membranproteine und Ionenkanäle einlagern und deren Reaktion auf Botenstoffe untersuchen, wenn dadurch die Leitfähigkeit der Membran beeinflusst wird. Insbesondere mit Ionenkanälen lassen sich so gezielt Biosensoren bauen.

In den Ersatzschaltbildern der Abbildungen 3 bis 5 ist der Widerstand/die Impedanz in der elektrisch leitfähigen Flüssigkeit (Elektrolyt) außerhalb des Spalts der Übersicht halber nicht eingezeichnet.

Das gemessene Rauschen setzt sich aus dem Rauschen der Messvorrichtung und dem Rauschen der Abdichtimpedanz der zu untersuchenden Substanz zusammen. Das Rauschen der Messvorrichtung kann gegebenenfalls durch Vergleichsmessungen ohne Substanz auf dem Träger eliminiert werden.

In einer weiteren bevorzugten Ausführungsform umfasst das erfindungsgemäße Verfahren in Schritt (c):

(c1) Bestimmen der Impedanz, vorzugsweise des Realteils der Impedanz, aus der Rauschspannung, und
(c2) Bestimmen der Abdichtung der Substanz auf dem Träger aus der Impedanz, vorzugsweise aus dem Realteil der Impedanz.

Stärker bevorzugt wird in Schritt (c1) dieser Ausführungsform die Impedanz, vorzugsweise der Realteil der Impedanz, aus der zeitabhängigen Rauschspannung bestimmt.

Der Realteil der Impedanz kann z.B. gemäß

$$S_V(f) = 4k_B T \cdot \mathrm{Re}(Z) \hspace{4cm} \text{(IV)}$$

aus der spektralen Leistungsdichte $S_V(f)$ bestimmt werden.

In noch einer weiteren bevorzugten Ausführungsform umfasst das erfindungsgemäße Verfahren in Schritt (c):

(c1) Bestimmen der spektralen Leistungsdichte aus der Rauschspannung, und
(c2) Bestimmen der Abdichtung der Substanz auf dem Träger aus der spektralen Leistungsdichte.

Stärker bevorzugt wird in Schritt (c1) dieser Ausführungsform die spektrale Leistungsdichte aus der zeitabhängigen Rauschspannung $V(t)$ bestimmt.

Zur Beschreibung von elektrischem Rauschen wird in dieser Ausführungsform die gut geeignete spektrale Leistungsdichte verwendet. Für eine gegebene zeitabhängigen Größe $a(t)$ kann man deren spektrale Leistungsdichte (auch "Leistungsdichtespektrum" oder einfach "Leistungsdichte") $S_a(f)$ definieren als:

$$S_a(f) := \lim_{T\to\infty} \frac{1}{2T} \left| \int_{-T}^{T} e^{i2\pi ft} a(t)\, dt \right|^2 \qquad (V)$$

Für die spektrale Leistungsdichte des Rauschens der Gesamtimpedanz gilt

$$S_V(f) = 4kT\cdot\mathrm{Re}\ (Z_{gesamt})$$
$$= 4kT\cdot\mathrm{Re}\ (Z_{erste\ Elektrode} + Z_{Flüssigkeit} + Z_{Abdichtung} + Z_{zweite\ Elektrode}) \qquad (VI)$$
$$= 4kT\cdot(\mathrm{Re}\ (Z_{erste\ Elektrode}) + \mathrm{Re}\ (Z_{Flüssigkeit}) + \mathrm{Re}\ (Z_{Abdichtung}) + \mathrm{Re}\ (Z_{zweite\ Elektrode}))$$

Das Rauschen und seine Messung wird also von einer imaginären Impedanz (also ohne Realteil), wie sie z.B. bei rein kapazitiven Elektroden auftritt, vorteilhafterweise nicht beeinflusst. Eine imaginäre Impedanz geht in die Messung nicht ein und stört diese demnach nicht. Damit können kapazitive Elektroden, deren Impedanz fast ausschließlich imaginär ist, zum Einsatz im erfindungsgemäßen Verfahren verkleinert werden (bis an die Grenzen des technisch möglichen), da der mit der Verkleinerung einhergehende Anstieg der imaginären Impedanz die Messung nicht stört.

Für die Ermittlung der spektralen Leistungsdichte wird der zeitliche Mittelwert

$$\bar{a} := \lim_{T\to\infty} \frac{1}{2T} \int_{-T}^{T} a(t)\, dt \qquad (VII)$$

bevorzugt ignoriert bzw. auf Null gesetzt, indem die allgemeine Funktion $a(t)$ in ihren zeitlichen Mittelwert und ihre Schwankung aufgeteilt wird und indem bei der Behandlung des Rauschens nur die Schwankung herangezogen wird. Sofern der zeitliche Mittelwert von Null verschieden ist, erscheint er in der spektralen Leistungsdichte als Delta-Funktion bei der Frequenz Null. Durch Ignorieren bzw. auf Null setzen kann die mathematisch aufwändige Behandlung einer derartigen Delta-Funktion vermieden werden.

In dieser Ausführungsform ist es bevorzugt, dass in Schritt (c2) die Abdichtung aus dem Integral der spektralen Leistungsdichte über einen vorbestimmten Frequenzbereich $f_1$ bis $f_2$ oder/und aus der Wurzel des Integrals der spektralen Leistungsdichte über einen vorbestimmten Frequenzbereich $f_1$ bis $f_2$ bestimmt wird:

$$V_{rms} := \sqrt{\int_{f_1}^{f_2} S_V(f)\, df} \qquad (VIII)$$

Die Wurzel des Integrals der spektralen Leistungsdichte über einen vorbestimmten Frequenzbereich $f_1$ bis $f_2$ wird auch $V_{rms}$-Wert genannt (siehe Gleichungen (I) und (II)). Es wird bevorzugt, dass der $V_{rms}$-Wert bestimmt wird, indem man aus dem Rauschspannungssignal einen vorbestimmten Frequenzbereich $f_1$ bis $f_2$ filtert, z.B. über einen Bandpass zur Wahl des interessierenden Frequenzbereichs, und indem man dann den Effektivwert des Quadrats der Spannung in einem vorbestimmten Zeitintervall bildet.

In einer weiteren bevorzugten Ausführungsform umfasst das erfindungsgemäße Verfahren in Schritt (c):

(c1) Bestimmen der Autokorrelationsfunktion aus der Rauschspannung, und
(c2) Bestimmen der Abdichtung aus der aus dem in Schritt (c1) erhaltenen Autokorrelationsfunktion.

In dieser Ausführungsform der vorliegenden Erfindung wird zur Rauschanalyse die so genannte Autokorrelationsfunktion benutzt, die für eine zeitabhängige Größe $a(t)$ als

$$\rho(\tau) := \lim_{T\to\infty} \frac{1}{2T} \int_{-T}^{T} a(t)a(t+\tau)\, dt \qquad (IX)$$

definiert ist, die eine andere Darstellung der spektralen Leistungsdichte ist. Die spektrale Leistungsdichte ist die Fouriertransformation der Autokorrelationsfunktion (und umgekehrt). Es ist stärker bevorzugt, dass in Schritt (c1) die Autokorrelationsfunktion aus der zeitabhängigen Rauschspannung *V(t)* bestimmt wird.

Im erfindungsgemäßen Verfahren können die Messungen beliebig lang sein. Typischerweise wird das Rauschen der Impedanz über Zeiten von einigen Millisekunden bis einige Minuten analysiert. Bevorzugt beträgt die Messzeit 10 ms bis 1000 s, stärker bevorzugt 1 bis 300 s, noch stärker bevorzugt 5 bis 60 s, am stärksten bevorzugt 10 bis 30 s.

Beispiel 1 zeigt, dass durch das erfindungsgemäße Verfahren Änderungen der Impedanz in Echtzeit beobachtet werden können. Das Beispiel zeigt einen Impedanzanstieg innerhalb weniger Millisekunden und einen ebenso schnellen Abfall, der durch eine Vergrößerung bzw. Verringerung des Rauschens in entsprechender Geschwindigkeit demonstriert wurde.

In einer weiteren bevorzugten Ausführungsform detektiert das erfindungsgemäße Verfahren daher schnelle Impedanzänderungen. Insbesondere detektiert das erfindungsgemäße Verfahren Impedanzänderungen mit einer Auflösung von höchstens 1000 ms, stärker bevorzugt höchstens 100 ms, noch stärker bevorzugt höchstens 10 ms, am stärksten bevorzugt höchstens 1 ms.

In noch einer weiteren bevorzugten Ausführungsform wird die Detektion schneller Impedanzänderungen mit einer parallelen Messung an einer Mehrzahl von Elektroden, wie z.B. unten beschrieben, kombiniert.

Das aus der Rauschspannung erhaltene Leistungsdichtespektrum erstreckt sich über einen weiten Frequenzbereich und liefert anhand seines Verlaufs mehr Informationen als die Messung der Impedanz bei einer einzelnen Frequenz. Im erfindungsgemäßen Verfahren kann man mit der spektralen Leistungsdichte der Rauschspannung vorteilhafterweise alle Frequenzen parallel untersuchen.

Der Frequenzbereich, über den die Rauschspannung im vorliegenden Verfahren gemessen werden kann, ist nur durch die Eigenschaften der verwendeten Vorrichtungen beschränkt. Insbesondere wird durch die Eigenschaften der Messvorrichtung die maximal mögliche zeitliche Auflösung des erfindungsgemäßen Verfahrens bestimmt.

In der Praxis kann sowohl im niederfrequenten (unterhalb von etwa 300 Hz) als auch im hochfrequenten Bereich (oberhalb von etwa 30.000 Hz) die spektrale Leistungsdichte vom Elektrodenrauschen dominiert sein. Im Bereich von etwa 300 bis etwa 30.000 Hz zeigt sich ein durch die Abdichtung einer Substanz auf einem Träger hervorgerufenes zusätzliches Rauschen. Daher wird im erfindungsgemäßen Verfahren das Rauschen im Frequenzbereich $f_1$ bis $f_2$ vorzugsweise von etwa 100 Hz bis etwa 100 kHz oder in einem Teilbereich hiervon, stärker bevorzugt von etwa 300 Hz bis etwa 30 kHz oder in einem Teilbereich hiervon, noch stärker bevorzugt von etwa 2 kHz bis etwa 10 kHz oder in einem Teilbereich hiervon bestimmt.

Das Integral der spektralen Leistungsdichte über den Frequenzbereich $f_1$ bis $f_2$ von etwa 2 bis etwa 10 kHz kann sehr gut mit der Abdeckung durch die abdichtende Substanz auf dem Träger korrelieren. Bevorzugt wird daher das Integral der spektralen Leistungsdichte über die Grenzen $f_1$ bis $f_2$ von etwa 100 Hz bis etwa 100 kHz oder einem Teilbereich hiervon, stärker bevorzugt über etwa 300 Hz bis etwa 30 kHz oder einem Teilbereich hiervon, noch stärker bevorzugt über etwa 2 kHz bis etwa 10 kHz oder einem Teilbereich hiervon bestimmt.

Die Schritte (b) und (c) des erfindungsgemäßen Verfahrens können mit zeitlichem Abstand zueinander ausgeführt werden.

In Schritt (d) einer bevorzugten Ausführungsform des erfindungsgemäßen Verfahrens wird aus der Abdichtung der Substanz auf dem Träger die Adhäsion der Substanz auf dem Träger bestimmt. Die Adhäsion einer Substanz kann insbesondere für Substanzen bestimmt werden, die zur selbständigen Anlagerung auf einen Träger fähig sind. Substanzen, die zur selbständigen Anlagerung fähig sind, sind insbesondere biologische Substanzen wie z.B. Zellen, Zellschichten, Lipidmembranen, etc., wie unten beschrieben.

Im Allgemeinen steigt die Adhäsion einer sich selbständig anlagernden Substanz mit steigender Abdichtung an. Daher können die oben beschriebenen Parameter, die ein Maß für die Abdichtung sein können, auch ein Maß für die Adhäsion der Substanz auf dem Träger sein.

Zur Durchführung von Schritt (b), Schritt (c) oder/und gegebenenfalls Schritt (d) des erfindungsgemäßen Verfahrens können geeignete analoge elektronische Vorrichtungen verwendet werden, die dem Fachmann bekannt sind (Filter, Vorrichtungen zur Integration, Fouriertransformation, Bestimmung von $V_{rms}$, etc.). Schritt (b) oder/und Schritt (c) des erfindungsgemäßen Verfahrens kann auch mit einer Kombination von Computer(n) und einer analogen Vorrichtungen/analogen Vorrichtungen ausgeführt werden. Der/die Computer umfasst/umfassen einen zur Durchführung bzw. Steuerung der Verfahrensschritte geeigneten Programmcode. Anhand der Angaben dieser Beschreibung kann der Fachmann einen geeigneten Programmcode erstellen.

Die mit dem erfindungsgemäßen Verfahren zu untersuchende Substanz kann eine biologische Substanz, insbesondere eine Zelle, ein Zellverband (z.B. ein Gewebe), eine Zellkultur oder eine Membran sein. Es kann jede Zelle, jeder Zellverband, jede Zellkultur oder jede Membran untersucht werden, die auf ein Trägermaterial, welches eine oder mehrere Elektroden umfasst, aufgebracht werden kann, so dass die Substanz mindestens eine Elektrode ganz oder teilweise bedeckt.

Bevorzugt wird die Adhäsion einer Zelle, eines Zellverbandes oder einer Zellkultur bestimmt, d.h. es wird bevorzugt die Zelladhäsion bestimmt.

Bevorzugt bildet der Zellverband oder die Zellkultur eine Ein-Zell-Schicht, insbesondere eine konfluente Ein-Zell-Schicht, oder eine Mehr-Zell-Schicht. Ein Beispiel für eine geeignete Zelllinie ist die gut etablierte Epithelzelllinie MDCK. Es handelt sich dabei um Zellen aus der Hundeniere (die Buchstaben CK stehen für ‚canine kidney'). Von MDCK sind verschiedene Linien bekannt, die sich vor allem in der Dichtigkeit der gebildeten Zellschicht unterscheiden. Alle diese Linien sind für das erfindungsgemäße Verfahren geeignet.

In einer weiteren bevorzugten Ausführungsform wird die Zelle, die Zellkultur oder der Zellverband auf dem Träger kultiviert.

Die Substanz kann ebenso eine künstliche Lipidschicht (Lipiddoppelmembran) sein, die gegebenenfalls Proteine enthält.

Der Träger für die Substanz ist aus einem Material gefertigt, das geeignet ist, die mindestens eine erste und gegebenenfalls die zweite Elektrode einzubetten. Es handelt sich dabei um einen elektrischen Isolator. In einer bevorzugten Ausführungsform wird der Träger aus einem Halbleiter, z.B. Silizium, GaAs oder GaN (insbesondere wenn als erste Elektrode(n) Transistoren verwendet werden), Glas, einem Polymer, Saphir oder/und Verbundwerkstoffen aus diesen Materialien gebildet. Derartige Trägermaterialien sind dem Fachmann bekannt.

In einer weiteren bevorzugten Ausführungsform ist der Träger zur Aufnahme biologischer Materialien geeignet, d.h. der Träger ist biokompatibel. Hierzu sind seine Oberflächeneigenschaften von besonderer Bedeutung. In einer besonders bevorzugten Ausführungsform ist die Oberfläche durch eine geeignete Beschichtung derart verändert, dass das Aufbringen, Wachstum oder die Adhäsion von Zellen auf der Beschichtung gefördert wird. Bevorzugte Beschichtungen bestehen aus Polypeptiden wie Laminin, Polylysin, Collagen oder/und Fibronectin. Eine dünne Beschichtung wie etwa Polylysin oder/und Kollagen erleichtert das Anwachsen und Kultivieren von Zellen oder/und Zellschichten auf dem Träger, verändert aber die elektrischen Eigenschaften der Elektrode nicht wesentlich. Dem Fachmann sind biokompatible Oberflächen und Beschichtungsmaterialien bekannt.

Im erfindungsgemäßen Verfahren werden bevorzugt Elektroden eingesetzt, die ihre elektrischen Eigenschaften während des Messzeitraumes im Wesentlichen nicht verändern.

Im Allgemeinen entspricht die Zusammensetzung der leitfähigen Flüssigkeit im Spalt zwischen der Substanz und der mindestens einen ersten Elektrode während der Messdauer im Wesentlichen der Zusammensetzung der restlichen leitfähigen Flüssigkeit. Es kann aber auch z.B. bei auf dem Träger wachsenden Epithelzellen zwischen Apikal- und Basalseite ein Konzentrationsgradient aufgebaut werden, d.h. im Spalt zwischen Zellschicht und Substrat kann eine An- oder Abreicherung bestimmter Stoffe, insbesondere Ionen, erfolgen, wodurch der Widerstand $r_s$ und damit die Abdichtimpedanz beeinflusst wird. Mit dem erfindungsgemäßen Verfahren kann also auch ein durch die Zellen bewirkter Konzentrationsaufbau beobachtet werden.

Die Elektrodenimpedanz ist die Impedanz, die bei der Ankopplung der auf Elektronenleitung (in Metallen) oder Elektronen/Löcher-Leitung (in Halbleitern) basierenden Ladungstransports an den auf Ionenleitung basierenden Ladungstransport im Elektrolyten entsteht. Sie hängt von der Bauform (z.B. blanke Metallelektrode, Silber-Silberchlorid-Elektrode, Feldeffekttransistor) und der Größe der Elektroden ab.

Im erfindungsgemäßen Verfahren kann die mindestens eine erste Elektrode umfasst von einem Träger eine spannungssensitive Elektrode sein. Dem Fachmann ist bekannt, wie eine solche spannungssensitive Elektrode aufgebaut ist. In einer bevorzugten Ausführungsform ist die mindestens eine erste Elektrode ein Transistor, insbesondere ein Feldeffekttransistor, oder eine Metallelektrode. Bevorzugte Materialien für eine erfindungsgemäße Metallelektrode werden ausgewählt aus Edelmetallen wie Platin, Gold, Titan und Legierungen davon, Indium-Zinn-Oxid (ITO), und Halbleitern wie Silizium, Germanium, GaAs, GaN und Legierungen davon.

Der Kontakt zwischen der mindestens einen ersten Elektrode und der Substanz kann direkt oder durch einen dünnen elektrischen Isolator, z.B. Siliziumdioxid, ausgeführt sein.

Bei den erfindungsgemäßen Elektroden bestimmt der Imaginäranteil die Impedanz, und der Realteil der Impedanz kann im Allgemeinen vernachlässigt werden. Eine Verkleinerung der Elektrodenfläche bewirkt also nur den Anstieg der imaginären Impedanz (siehe oben). Im erfindungsgemäßen Verfahren und der erfindungsgemäßen Vorrichtung (siehe unten) beeinflusst die imaginäre Impedanz der mindestens einen ersten Elektrode die Messung der Rauschspannung nicht, d.h. kleine Elektroden stellen für das erfindungsgemäße Verfahren kein Problem dar. Bei niedrigen Rauschfrequenzen, bei denen der Imaginärteil der Impedanz im Vergleich zum Imaginärteil der Impedanz bei höheren Frequenzen größer ist, macht sich diese Eigenschaft des erfindungsgemäßen Verfahrens/der erfindungsgemäßen Vorrichtung besonders vorteilhaft bemerkbar.

Das erfindungsgemäße Verfahren ist demnach geeignet, um mit kleinen Sensorflächen zu arbeiten. Eine untere Grenze für die Elektrodengröße ist nur durch die technischen Möglichkeiten zur Herstellung von Elektroden umfasst von einem Träger gegeben. Insbesondere können vorteilhafterweise Elektroden eingesetzt werden, deren Durchmesser unterhalb des Durchmessers von Zellen (typischerweise 10 bis 20 $\mu$m) liegen, die auf dem Träger kultiviert werden, bzw. bei denen die von der Elektrode auf dem Träger bedeckte Fläche kleiner als die von der Zelle bedeckte Fläche ist.

Es ist bevorzugt, dass die mindestens eine erste Elektrode, insbesondere eine runde Elektrode, einen Durchmesser von mindestens 1 $\mu$m, stärker bevorzugt mindestens 2 $\mu$m, und noch stärker bevorzugt von mindestens 5 $\mu$m

hat. Es ist ferner bevorzugt, dass die mindestens eine erste Elektrode einen Durchmesser von höchstens 100 $\mu$m, stärker bevorzugt von höchstens 30 $\mu$m, am stärksten bevorzugt höchstens 10 $\mu$m hat.

Die Elektrode kann eine beliebige Form haben, z.B. rund, viereckig, etc. Die Form der Elektrode ist nur durch die technischen Möglichkeiten zur Herstellung von Elektroden umfasst von einem Träger beschränkt. Transistoren können z.B. mit rechteckigen Gateflächen hergestellt werden, wobei typischerweise die Gatelänge (in Stromflussrichtung) deutlich größer als die Gatebreite ist. Die mindestens eine erste Elektrode bedeckt bevorzugt eine Fläche des Trägers von mindestens 1 $\mu$m$^2$, stärker bevorzugt mindestens 5 $\mu$m$^2$, noch stärker bevorzugt mindestens 25 $\mu$m$^2$. Die mindestens eine erste Elektrode bedeckt bevorzugt eine Fläche des Trägers von höchstens 10 000 $\mu$m$^2$, stärker bevorzugt höchstens 1 000 $\mu$m$^2$, noch stärker bevorzugt höchstens 100 $\mu$m$^2$.

Es ist ferner bevorzugt, dass der Träger eine Mehrzahl von ersten Elektroden umfasst, so dass die Abdichtimpedanz der auf den Träger aufgebrachten Substanz an einer Mehrzahl von Positionen gemessen werden kann. Da vorteilhafterweise sehr kleine Elektroden verwendet werden können, sind sehr hohe Elektrodendichten möglich. Die Elektroden können zum Beispiel linear angeordnet sein (siehe Beispiele) oder in Form eines zweidimensionalen Arrays. Der Abstand der Elektroden kann beliebig gewählt werden. Bevorzugt ist ein Abstand von höchstens 200 $\mu$m, stärker bevorzugt höchstens 50 $\mu$m, noch stärker bevorzugt höchstens 10 $\mu$m, am stärksten bevorzugt höchstens 2 $\mu$m. Der Träger umfasst bevorzugt mindestens 5, stärker bevorzugt mindestens 10, noch stärker bevorzugt mindestens 50, am stärksten bevorzugt mindestens 100 Elektroden. Der Träger umfasst bevorzugt maximal 20 000, stärker bevorzugt maximal 5 000, noch stärker bevorzugt maximal 1 000, am stärksten bevorzugt maximal 200 Elektroden.

Durch die Mehrzahl von Elektroden, an denen parallel gemessen wird, können auch Veränderungen der Substanz, z.B. Wanderungs-, Wachstums- oder/und Teilungsprozesse von Zellen auf dem Träger, beobachtet werden. Beispiel 2 zeigt z.B. eine Messung, die die hohe räumliche Auflösung einer Anordnung der ersten Elektroden nutzt, um ein Abdichtungsprofil einer einzelnen Zelle auf einem Träger zu erstellen.

Im erfindungsgemäßen Verfahren kann als zweite Elektrode jede Elektrode eingesetzt werden, die als Referenzelektrode in einer elektrisch leitfähigen Flüssigkeit geeignet ist. Insbesondere kann die zweite Elektrode größer als die mindestens eine erste Elektrode sein. Die zweite Elektrode kann auch wie die mindestens eine erste Elektrode beschaffen sein oder kann auch eine Silberelektrode, insbesondere eine Ag/AgCl-Elektrode, sein.

Es ist bevorzugt, dass die mindestens eine erste Elektrode und die zweite Elektrode so beschaffen sind, dass deren Impedanz gegenüber der Abdichtimpedanz vernachlässigt werden kann.

Die Leitfähigkeit der elektrisch leitfähigen Flüssigkeit kann in weiten Grenzen variieren. Die elektrisch leitfähige Flüssigkeit kann eine wässrige Salzlösung sein, insbesondere ein Zellkulturmedium oder eine Salzlösung, die zur Haltung von Zellen geeignet ist. Dem Fachmann sind geeignete Zellkulturmedien und Salzlösungen bekannt.

Für nichtbiologische Anwendungen kann der gesamte Bereich von Flüssigkeiten mit sehr niedriger Leitfähigkeit (wie z.B. deionisiertes Wasser) bis zu Flüssigkeiten mit sehr hoher Leitfähigkeit (z.B. eine gesättigte wässrige Salzlösung) genutzt werden. Bei gegebener Spaltgeometrie kann die Abdichtimpedanz durch geeignete Wahl der Leitfähigkeit der elektrisch leitfähigen Flüssigkeit in einen gut messbaren Bereich gebracht werden.

Es ist bevorzugt, dass die Impedanz der elektrisch leitfähigen Flüssigkeit gegenüber der Abdichtimpedanz vernachlässigt werden kann.

Die Impedanz der ersten Elektrode, der zweiten Elektrode oder/und der elektrisch leitfähigen Flüssigkeit kann vernachlässigt werden, wenn sie höchstens 10 %, vorzugsweise höchstens 1 %, stärker bevorzugt höchstens 0,1 %, am stärksten bevorzugt höchstens 0,01 % der Abdichtimpedanz beträgt. Gegebenenfalls kann die Impedanz der ersten Elektrode, der zweiten Elektrode oder/und der elektrisch leitfähigen Flüssigkeit durch eine Kontrollmessung ohne Substanz auf dem Träger gesondert bestimmt werden.

Die Abdichtimpedanz ist über die Spaltbreite und Abdichtgeometrie mit der Leitfähigkeit der elektrisch leitfähigen Flüssigkeit verknüpft. Man kann also z.B. aus der bekannten Leitfähigkeit der elektrisch leitfähigen Flüssigkeit und der gemessenen Abdichtimpedanz die Spaltbreite berechnen.

Die Impedanz der elektrisch leitfähigen Flüssigkeit ist die Impedanz, die aufgrund der endlichen Leitfähigkeit des Elektrolyten zwischen erster und zweiter Elektrode entsteht und die proportional zur Leitfähigkeit des Elektrolyten ist. Der Proportionalitätsfaktor ist allein von der Geometrie der Anordnung aus erster Elektrode, zweiter Elektrode und elektrisch leitfähiger Flüssigkeit bestimmt. Falls die erste Elektrode klein gegen die Abmessungen der zweiten Elektrode und des Bades enthaltend die elektrisch leitfähige Flüssigkeit ist, wird der Proportionalitätsfaktor für die elektrisch leitfähige Flüssigkeit von der Geometrie der ersten Elektrode bestimmt. Zum Beispiel beträgt die Impedanz eines Elektrolyten bei einer kreisförmigen ebenen Elektrode von Durchmesser d, die sich einseitig in Kontakt mit einem unendlich ausgedehnten Elektrolyten (Halbraum) befindet,

$$R_{Bad} = \frac{\rho}{2d} \qquad\qquad\qquad (X).$$

Dabei ist P der spezifische Widerstand des Elektrolyten und wird in Ohm·cm angegeben. Dieser Wert ist auch für die in der Praxis häufig anzutreffenden quadratischen Elektroden eine gute Näherung, wenn man die quadratische Elektrode in eine kreisförmige Elektrode mit der gleichen Oberfläche umrechnet.

Die Abdichtimpedanz hängt von der Breite des Spalts zwischen Träger und Substanz auf dem Träger ab. Die Spaltbreite kann wenige Nanometer betragen (z.B. bei mechanisch unterstütztem Kontakt sehr glatter Oberflächen oder bei Anlagerung einer Lipidmembran). Für den aktiven Kontakt der Substanz mit dem Träger (wie z.B. in Beispiel 1) beträgt die Spaltbreite bevorzugt 0 bis etwa 5 nm. Für eine Lipidmembran auf einem Träger (wie z.B. in Beispiel 4) beträgt die Spaltbreite bevorzugt 0 bis etwa 10 nm. Bei biologischen Zellen, die auf der Elektrode adhäriert sind, kann die Spaltbreite im Bereich von etwa 50 nm liegen. Ist die Substanz eine biologische Zelle, eine Zellschicht, eine Zellkultur oder ein Zellverband, beträgt die Spaltbreite bevorzugt mindestens etwa 20 bis etwa 200 nm, stärker bevorzugt mindestens etwa 30 bis etwa 100 nm.

Es ist bevorzugt, dass der Spalt klein gegen den Durchmesser der mindestens einen ersten Elektrode ist. Wären Spaltbreite und Durchmesser der Elektrode von vergleichbarer Größe, würde es zu keiner signifikanten Erhöhung der Abdichtimpedanz kommen.

Als Anwendungen für das erfindungsgemäße Verfahren kommen z.B. in Betracht: Die Untersuchung der spontanen Zelladhäsion auf einem Substrat, der Einfluss von spezifischen Beschichtungen auf die Adhäsion, die Entwicklung der Adhäsion mit zunehmender Kulturdauer, der Einfluss zugegebener Pharmaka auf die Adhäsion. Ein großer Vorteil ist, dass sich - aufgrund der kleinen Elektroden- bzw. Transistorgröße - einzelne Zellen untersuchen lassen. Man erhält nicht ein gemitteltes Signal vieler Zellen, sondern kann die Anlagerungs- und Ablöseprozesse einer einzelnen Zelle untersuchen, die typischerweise von ganz bestimmten Signalwegen innerhalb der Zelle ausgelöst werden. Da die Zelle als ganzes reagiert, unterschiedliche Zellen diesen Signalweg jedoch zu unterschiedlichen Zeitpunkten anstoßen, gehen bei einer Mittelung über viele Zellen Informationen verloren.

Ein weiterer Gegenstand der vorliegenden Erfindung ist eine Vorrichtung zur Durchführung des oben beschriebenen erfindungsgemäßen Verfahrens gemäß Ansprüche 24-27.

In einer bevorzugten Ausführungsform umfasst die erfindungsgemäße Vorrichtung weiterhin

(iv) Mittel zum Bestimmen der Adhäsion der Substanz auf dem Träger aus der Abdichtung der Substanz auf dem Träger.

Der Träger gemäß (i) kann ein Träger sein, wie er für das erfindungsgemäße Verfahren oben beschrieben ist. Es ist bevorzugt, dass der Träger eine Mehrzahl von ersten Elektroden umfasst.

Das Mittel zum Messen des Rauschens gemäß (ii) kann Mittel zur Ausführung des Schrittes (b) des erfindungsgemäßen Verfahrens, wie z.B. oben beschrieben, umfassen. Es ist eine Vorrichtung bevorzugt, mit der eine strom- und spannungslose Messung wie oben beschrieben, durchgeführt werden kann. Diese Vorrichtung wird bevorzugt durch einen Computer gesteuert und umfasst Mittel zum Speichern der Ergebnisse der Messung des Rauschens.

Das Mittel zur Bestimmung der Abdichtung auf dem Träger aus dem elektrischen Rauschen gemäß (iii) kann Mittel zur Ausführung des Schrittes (c) des erfindungsgemäßen Verfahrens, wie z.B. oben beschrieben, umfassen.

Es ist bevorzugt, das das Mittel gemäß (iii) in Form eines auf einem Computer ausführbaren Programmprodukts bereitgestellt wird.

Das Mittel zur Bestimmung der Abdichtung auf dem Träger aus dem elektrischen Rauschen gemäß (iii) umfasst bevorzugt Mittel zum

(iii1) Bestimmen der spektralen Leistungsdichte aus der Rauschspannung, und
(iii2) Bestimmen der Abdichtung der Substanz auf dem Träger aus der spektralen Leistungsdichte.

Bevorzugt umfasst (iii1) Mittel, mit denen die spektrale Leistungsdichte aus der zeitabhängigen Rauschspannung bestimmt werden kann.

Bevorzugt umfasst (iii2) Mittel, um die Abdichtung aus dem Integral der spektralen Leistungsdichte über die Frequenz oder/und aus der Wurzel des Integrals der spektralen Leistungsdichte über die Frequenz bestimmen zu können.

Es ist ferner bevorzugt, dass die erfindungsgemäße Vorrichtung Mittel umfasst, mit denen das Rauschen bevorzugt im Bereich von etwa 100 Hz bis etwa 100 kHz oder in einem Teilbereich hiervon, stärker bevorzugt von etwa 300 Hz bis etwa 30 kHz oder in einem Teilbereich hiervon und am stärksten bevorzugt zwischen etwa 2 kHz und etwa 10 kHz oder in einem Teilbereich hiervon bestimmt werden kann.

Die erfindungsgemäße Vorrichtung kann so beschaffen sein, dass die Mittel (ii) und (iii) räumlich voneinander getrennt sind oder/und in zeitlichem Abstand angewendet werden können.

Das Mittel gemäß (iv) in einer bevorzugten Ausführungsform kann Mittel zur Ausführung des Schrittes (d) einer bevorzugten Ausführungsform des erfindungsgemäßen Verfahrens, wie z.B. oben beschrieben, umfassen.

Bekannte Vorrichtungen sind nicht zur Durchführung des oben beschriebenen erfindungsgemäßen Verfahrens geeignet. Man kann eine erfindungsgemäße Vorrichtung erhalten, wenn man bekannte Vorrichtungen, z.B. zur Durchführung des etablierten ECIS-Verfahrens, mit geeigneten ergänzenden Vorrichtungen oder/und Softwareprodukten kombiniert, was eine schnelle Marktrealisierung bzw. -akzeptanz des erfindungsgemäßen Verfahrens fördern kann. Insbesondere können Elektroden umfassende Träger zur Kultur von biologischen Systemen, die bereits für das ECIS-Verfahren verwendet wurden, im erfindungsgemäßen Verfahren/in der erfindungsgemäßen Vorrichtung eingesetzt werden, sofern für den beabsichtigten Zweck nicht kleinere Elektroden notwendig sind.

Ein weiterer Gegenstand der vorliegenden Erfindung ist ein Verfahren zur Identifizierung einer Verbindung, die die Abdichtung einer Substanz auf einem Träger moduliert, umfassend die Schritte:

(I) Bestimmen der Abdichtung der Substanz auf einem Träger mit dem oben beschriebenen erfindungsgemäßen Verfahren oder/und mit der oben beschriebenen erfindungsgemäßen Vorrichtung in Abwesenheit der zu untersuchenden Verbindung,

(II) Bestimmen der Abdichtung der Substanz auf einem Träger mit dem oben beschriebenen erfindungsgemäßen Verfahren oder/und mit der oben beschriebenen erfindungsgemäßen Vorrichtung in Anwesenheit der zu untersuchenden Verbindung und

(III) Auswählen einer Verbindung, die die Abdichtung der Substanz auf dem Träger auf vorbestimmte Weise moduliert.

Ferner betrifft die Erfindung ein Verfahren zur Identifizierung einer Verbindung, die die Adhäsion einer Substanz auf einem Träger moduliert, umfassend die Schritte:

(1) Bestimmen der Adhäsion der Substanz auf einem Träger mit dem oben beschriebenen erfindungsgemäßen Verfahren oder/und mit der oben beschriebenen erfindungsgemäßen Vorrichtung in Abwesenheit der zu untersuchenden Verbindung,

(2) Bestimmen der Adhäsion der Substanz auf einem Träger mit dem oben beschriebenen erfindungsgemäßen Verfahren oder/und mit der oben beschriebenen erfindungsgemäßen Vorrichtung in Anwesenheit der zu untersuchenden Verbindung und

(3) Auswählen einer Verbindung, die die Adhäsion der Substanz auf dem Träger auf vorbestimmte Weise moduliert.

"Auf vorbestimmte Weise moduliert" bedeutet, dass vorbestimmt wird, ob die Abdichtung oder/und Adhäsion sich gegenüber dem Kontrollwert (zu untersuchende Verbindung abwesend) erhöht, vermindert oder vom Kontrollwert abweichen soll. Hierbei wird gegebenenfalls eine Schwelle vorbestimmt, die über- oder unterschritten werden muss, damit eine Verbindung gemäß Schritt (III) bzw. (3) ausgewählt wird.

Bevorzugt ist die im Identifizierungsverfahren verwendete Substanz eine biologische Substanz.

Ist beispielsweise die Substanz eine konfluente Schicht aus Zellen, die untereinander durch tight junctions verbunden sind, so können mit dem Identifizierungsverfahren Wirkstoffe aufgefunden werden, die mit den Zellen oder/und den tight junctions interagieren und die die Impedanz der tight junctions verändern. Wirkstoffe, die beispielsweise die Impedanz der tight junctions erhöhen, können für die Krebstherapie eingesetzt werden, da sie durch die Stabilisierung der tight junctions eine Zellablösung aus einem Tumor (und damit eine Metastasenbildung) verhindern könnten, was die Gefährlichkeit von Tumoren senken kann.

Ist beispielsweise die Substanz eine einzelne Zelle, so können mit dem erfindungsgemäßen Identifizierungsverfahren Wirkstoffe identifiziert werden, die die Abdichtung oder/und Adhäsion der Zelle auf dem Träger beeinflussen.

Ist die Substanz beispielsweise eine Lipiddoppelmembran, so können in diese Membran Proteine, z.B. Ionenkanäle oder/und Ionenpumpen, eingebracht werden. Mithilfe des erfindungsgemäßen Identifizierungsverfahrens können Substanzen identifiziert werden, die die Leitfähigkeit der Lipidmembran beeinflussen, indem die Substanzen die Leitfähigkeit der eingebrachten Proteine, insbesondere von eingebrachten Ionenkanälen oder/und Ionenpumpen, beeinflussen.

Weiterhin kann das erfindungsgemäße Identifizierungsverfahren zur Diagnostik eingesetzt werden, indem Proben aus klinischen Isolaten, z.B. Isolaten aus Tumoren, auf ihre Fähigkeit zur Abdichtung oder/und Adhäsion auf Trägern untersucht werden. So kann beispielsweise die Abdichtung oder/und Adhäsion von kultiviertem Tumorgewebe eine quantitative Aussage über die Gutartigkeit bzw. Bösartigkeit des Tumors erlauben. Je höher die Abdichtung oder/und Adhäsion, desto geringer kann die Gefährlichkeit des Tumors eingeschätzt werden, da die Neigung zur Ablösung von Zellen und Zellverbänden aus dem Tumor geringer ist. Damit ist die Wahrscheinlichkeit von Metastasen geringer.

Durch die oben beschriebenen Anwendungen kann das erfindungsgemäße Identifizierungsverfahren ebenso zur Untersuchung der Mechanismen der Tumorentstehung eingesetzt werden.

In einer weiteren bevorzugten Ausführungsform wird das erfindungsgemäße Identifizierungsverfahren im High-Throughput-Format durchgeführt.

Ein weiteres nicht erfindungsgemäßes Beispiel ist ein Biosensor, umfassend einen Träger umfassend mindestens eine erste Elektrode und eine Lipiddoppelmembran, die die mindestens eine erste Elektrode ganz oder teilweise abdeckt. In der Lipiddoppelmembran können Proteine, z.B. Ionenkanäle enthalten sein. Diese Ionenkanäle können ligandengesteuert sein, so dass die Abdichtimpedanz der Lipiddoppelmembran durch das Vorhandensein von Liganden, die in einer Probe vorhanden sind, die mit dem Biosensor in Kontakt gebracht wird, moduliert wird. Geeignete Lipiddoppelmembranen, Ionenkanäle und Liganden sind dem Fachmann bekannt. Geeignete Träger und Elektroden sind im Zusammenhang mit dem erfindungsgemäßen Verfahren beschrieben. Die Modulation der Abdichtimpedanz kann durch dessen Rauschen, wie oben beschrieben, bestimmt werden.

Die Erfindung wird durch die folgenden Abbildungen und Beispiele weiter verdeutlicht.

**Abbildung 1** zeigt das erfindungsgemäße Messprinzip. Durch Messung der Rauschspannung als Impedanz Z zwischen Messelektrode und Elektrode in der elektrisch leitfähigen Flüssigkeit kann die Abdichtung/Adhäsion bestimmt werden.

**Abbildung 2:** Abdichtung durch perfekt isolierendes Objekt, schematisch.

**Abbildung 3:** Zelle auf Elektrode. Die Zellmembran weist eine hohe elektrische Impedanz auf und dichtet so die Elektrode ab.

**Abbildung 4:** Eine auf dem Substrat kultivierte Schicht aus Epithelzellen dichtet die Elektrode ab. Die Dichtigkeit der Gesamtschicht wird im Wesentlichen durch den Widerstand/die Impedanz der "tight junctions" zwischen den Zellen bestimmt.

**Abbildung 5:** Lipidmembran auf fester Unterlage ("supported lipid bilayer"). In die Membran können Transmembranproteine, insbesondere Ionenkanäle eingelagert sein, die die Abdichtimpedanz beeinflussen.

**Abbildung 6:** Silikonperle in Kontakt mit einem linearen Transistorarray. Rechts eine vergrößerte Ansicht, in der man den Kontaktbereich als leicht dunkleren Fleck mit einem hellen Rand erkennt. Er erstreckt sich über insgesamt 4 Transistoren und hat einen Durchmesser von ca. 50 $\mu$m.

**Abbildung 7:** Gatespannungen der Transistoren im Kontaktbereich. FETs 76 bis 79, die mit der Silikonperle in Kontakt sind, zeigen ein deutlich erhöhtes Rauschen. Zum besseren Verständnis ist die Gatefläche von FET 81 schwarz umrandet eingezeichnet. Sie hat eine Größe von ca. 6 x 7 $\mu$m$^2$. Die Gatefläche ist die wirksame, spannungsempfindliche Fläche eines Feldeffekttransistors. Sie entspricht der Elektrodenfläche bei gewöhnlichen Metallelektroden.

**Abbildung 8:** (a) Spektrale Leistungsdichten der Gatespannung von FET 77 ohne und mit Kontakt mit Silikonperle. (b) Die Differenz der Leistungsdichten von (a). Sie entspricht dem Rauschen, das durch die Abdichtimpedanz verursacht wird. Die durchgezogene Linie ist eine angefittete Funktion (siehe Beispiel 1). Der Konvention folgend sind in (a) und (b) die Quadratwurzeln der spektralen Leistungsdichten aufgetragen.

**Abbildung 9:** Der Kontakt wird nur kurz hergestellt, dann wieder aufgehoben. Der Frequenzbereich der gezeigten Transistorsignale ist 400 bis 10.000 Hz.

**Abbildung 10** zeigt eine adhärierte Zelle auf einem Siliziumchip mit Feldeffekttransistoren, links daneben die gatebezogen Spannungssignale der einzelnen Transistoren. Im Bereich der adhärierten Zelle ist das Rauschen stark erhöht.

**Abbildung 11** zeigt die spektralen Leistungsdichten des gatebezogenen Spannungsrauschens der Transistoren 104 bis 115. Der besseren Sichtbarkeit wegen sind die Leistungsdichten jeweils um den Faktor 1,3 versetzt, die Skala bezieht sich auf die unterste Kurve. Sowohl im niederfrequenten (unterhalb von etwa 300 Hz) als auch im hochfrequenten Bereich (oberhalb von etwa 30.000 Hz) ist die Leistungsdichte vom Transistorrauschen dominiert und für alle Transistoren gleich. Im Bereich von etwa 300 bis etwa 30.000 Hz zeigt sich das durch die Adhäsion hervorgerufene zusätzliche Rauschen.

**Abbildung 12:** Für die Charakterisierung der Impedanz der Abdichtung kann man z.B. eine Kenngröße $V_{rms}$ als die Wurzel des Integrals der spektralen Leistungsdichte von etwa 2 bis etwa 10 kHz definieren. Diese Kenngröße korreliert sehr gut mit der Abdeckung durch die adhärierte Zelle. Grafisch aufgetragen erhält man direkt das Profil der Abdichtung.

**Beispiel 1: Auf Oberfläche gedrückte Silikonperle**

Es wurden Siliziumchips mit Feldeffekttransistoren verwendet, wobei sowohl die spannungsempfindlichen Gatebereiche als auch der Rest des Chips von 10 nm dickem Siliziumdioxid bedeckt waren. Die verwendeten Transistoren hatten eine in etwa quadratische Gatefläche von etwa 6 $\mu$m) x 7 $\mu$m.

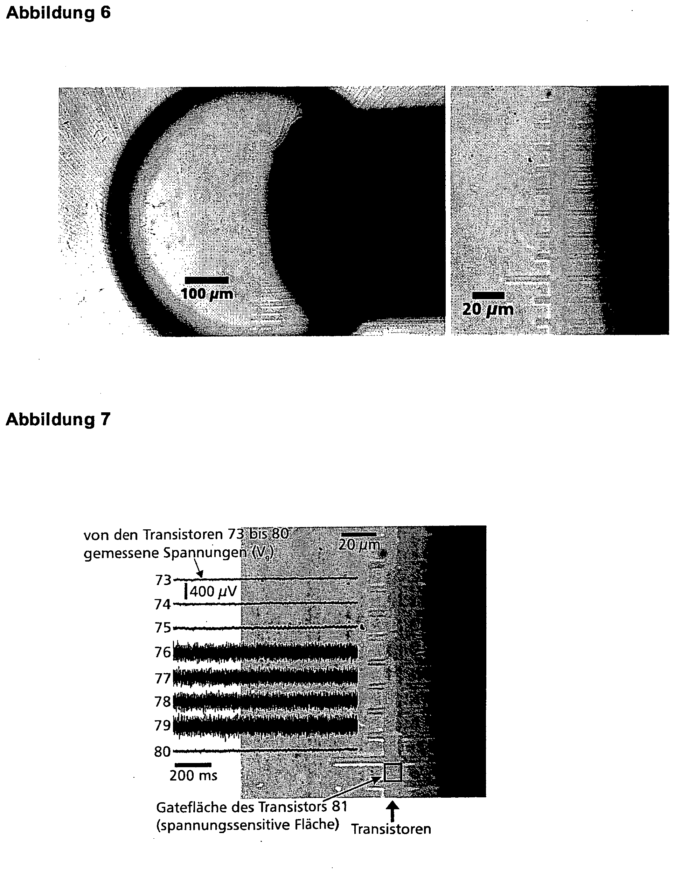

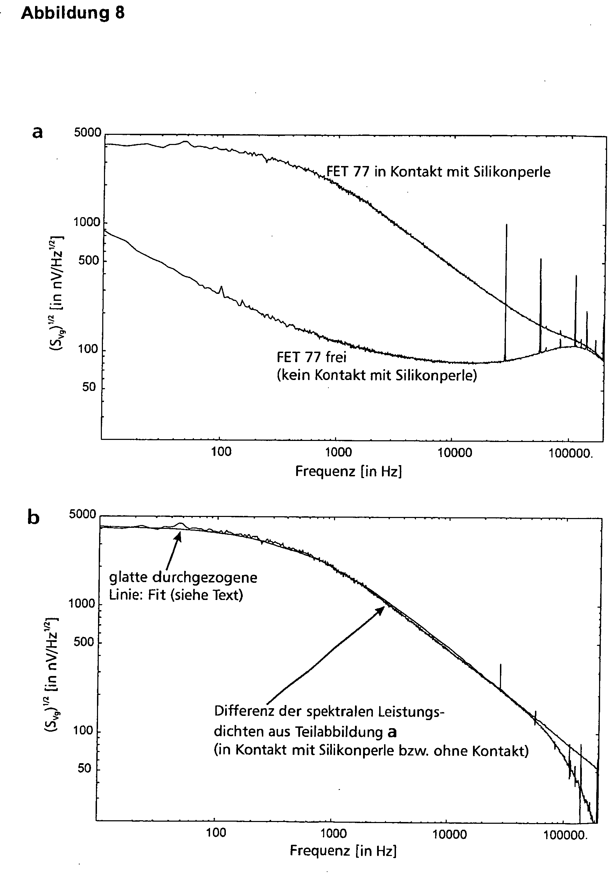

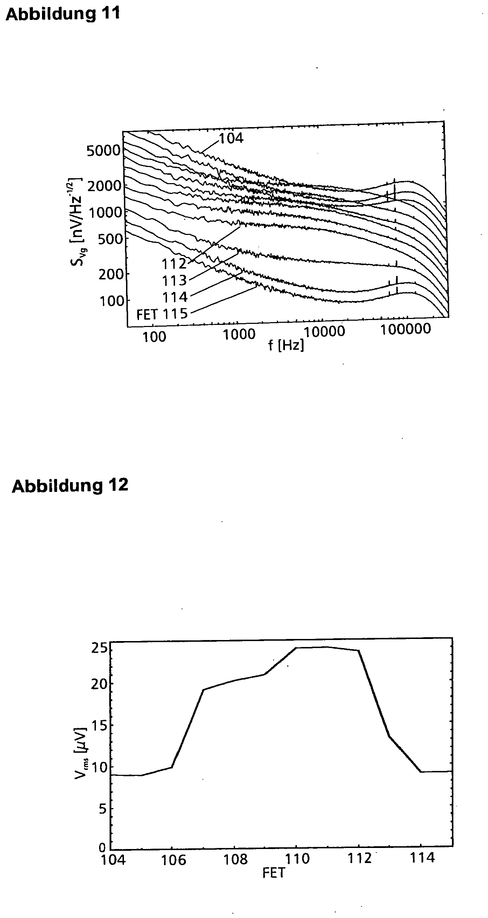

Der Drainstrom der Transistoren wurde mittels eines Strom-Spannungs-Wandlers, bestehend aus einem Operationsverstärker OPA627 und einem Rückkopplungswiderstand von 100 kOhm, in eine Spannung gewandelt, um einen Faktor 1000 verstärkt, mit einer Messkarte digitalisiert und von einem PC aufgezeichnet. Für die Aufnahme der in Abbildung 7, 9 und 10 gezeigten Spannungssignale wurde das Signal mit einem 10 kHz-Tiefpass gefiltert und mit 30 kSample/s aufgenommen. Bei den spektralen Leistungsdichten in Abbildung 8 und 11 wurde die volle Verstärkerbandbreite genutzt, was effektiv einem Tiefpass von 100 kHz entspricht, sowie eine Digitalisierungsrate von 1 MSample/s.

Wir betrachten den Fall eines isolierendes Objektes, das aktiv in Kontakt mit einem Transistor-Array gebracht wird. Abbildung 2 zeigt den schematischen Aufbau. Das Objekt isolierte den Spalt nach oben hin, so dass die Abdichtimpedanz aus der Leitfähigkeit des Spalts, der Kapazität des Substrats und der - so vorhanden - Leckleitfähigkeit des Substrats gebildet wurde.

Ein Experiment demonstriert diese Konstellation. Als isolierendes Objekt wurde eine in etwa kugelförmige Perle aus Silikon mit ca. 600 $\mu$m Durchmesser verwendet, die an der Spitze einer Kanüle montiert war und mittels eines fein steuerbaren Mikromanipulators über einen Siliziumchip mit zwei linearen Arrays aus Feldeffekttransistoren gebracht wurde (Abbildung 6). Als Elektrolyt diente normale elektrophysiologische Lösung mit einer Leitfähigkeit von 15 mS/cm.

Die Silikonperle wurde langsam bis auf die Transistoroberfläche abgesenkt, bis ein Kontakt entstand. Da die Silikonperle durchsichtig war, konnte man mit einem Mikroskop die durch den Kontakt entstehenden Newtonschen Interferenzen erkennen (Detailansicht in Abbildung 6).

Auf denjenigen Transistoren, die mit der Silikonperle in Kontakt standen und durch sie abgedichtet waren, zeigte sich ein stark erhöhtes Rauschen (Abbildung 7). Genauere Informationen lieferte die spektrale Leistungsdichte. Dazu wurden die spektralen Leistungsdichten des Rauschens von FET 77 im Kontaktfall und ohne Kontakt (d.h. mit wieder entfernter Silikonperle) aufgenommen (Abbildung 8a). Zur Analyse wurde die Differenz dieser Leistungsdichten gebildet (Abbildung 8b), denn diese Differenz entspricht genau dem Rauschen der Abdichtimpedanz. Sie ist bei niedrigen Frequenzen konstant und fällt oberhalb einer Grenzfrequenz mit $f^{3/2}$ ab. Dies ist der Verlauf, der von einer kontinuierlichen Beschreibung des Rauschens im Spalt vorhergesagt werden kann. Die durchgezogene Linie in Abbildung 8b ist ein Fit der Form

$$S_{fit}(f) = 4k_B T \cdot R \frac{1}{1 + (f/f_0)^{3/2}} \qquad\qquad (XI)$$

mit den gefitteten Werten

$$R = 1{,}1\,\mathrm{G}\Omega, \quad f_0 = 580\,\mathrm{Hz} \qquad\qquad (XII).$$

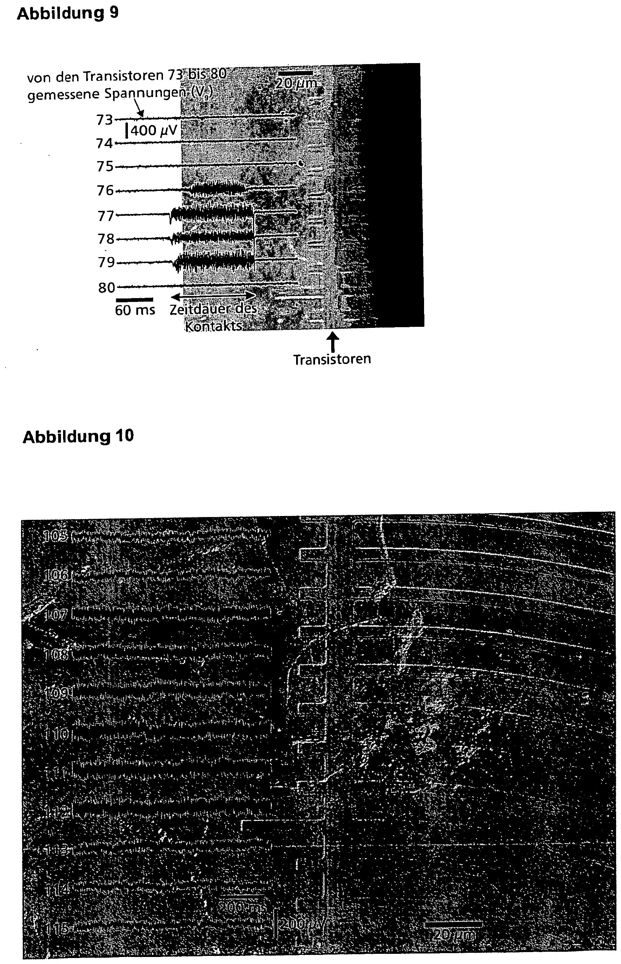

Die Ausbildung des Kontakts konnte in Echtzeit verfolgt werden. Dazu wurde die Silikonperle kurz in Kontakt mit dem Transistorarray gebracht und gleich wieder entfernt. Abbildung 9 zeigt, wie innerhalb von wenigen Millisekunden das Rauschen stark anstieg und auch wieder abfiel. Auch die zeitliche Ausbreitung des Kontaktbereichs ist zu erkennen: FETs 77 und 78 hatten als erste Kontakt, kurz danach FET 79 und mit deutlicher Verzögerung FET 76. Dies ist auch zu erwarten, da die von oben kommende Silikonperle zunächst nur mit ihrer 'Spitze' die Oberfläche berührte, dann bei weiterem Näherbringen platt gedrückt wurde, sich dadurch der Kontaktbereich vergrößert und die angrenzenden Transistoren Kontakt bekamen.

**Beispiel 2: Zelle auf Transistorarray**

Die Abdichtung kann von einer Zelle verursacht werden, die auf dem Substrat bzw. der Elektrode adhäriert ist. Abbildung 3 zeigt diese Situation schematisch. Die Zellmembran weist eine hohe Impedanz auf, so dass die Abdichtimpedanz in erster Linie von der Leitfähigkeit des Elektrolyten und der Breite des Spalts zwischen Zellmembran und Substratoberfläche bestimmt wird. Die Oberseite der unteren Zellmembran liegt auf dem von der Zelle eingestellten Ruhepotential des Zellinneren. Das Zellinnere steht wiederum über die obere Membran bzw. deren Leckleitfähigkeit und kapazitiver Leitfähigkeit mit dem umgebenden Elektrolyten in Kontakt.

Der in diesem Beispiel verwendete Messaufbau entsprach dem Messaufbau des Beispiels 1.

Abbildung 10 zeigt eine Zelle aus dem Hippocampus einer Ratte nach 16 Tagen in Kultur. Die Zelle ist auf dem Substrat (dem Chip) adhäriert. Die Transistoren, die von der Zelle bedeckt waren, zeigten ein erhöhtes Rauschen. Die spektralen Leistungsdichten in Abbildung 11 zeigen, dass das von der Abdichtung durch die Zelle hervorgerufene zusätzliche Rauschen im Bereich zwischen 300 und 30.000 Hz am besten zu detektieren ist. Um die Interpretation der Leistungsdichten etwas zu vereinfachen, kann man einen Frequenzbereich $[f_1, f_2]$ wählen und das darin enthaltene Rauschen in eine effektive Kenngröße $V_{rms}$ umrechnen, die die Dimension Volt hat:

$$V_{rms} := \sqrt{\int_{f_1}^{f_2} S_V(f)\, df} \qquad \text{(VIII)}.$$

In Abbildung 12 ist dies für die spektralen Leistungsdichten aus Abbildung 11 im Frequenzbereich 2 bis 10 kHz dargestellt. Man erhält ein Profil der Abdichtung.

Dieses Beispiel zeigt, wie das erfindungsgemäße Verfahren bzw. eine erfindungsgemäße Vorrichtung vorteilhaft mit Elektrodenarrays mit hoher räumlicher Auflösung eingesetzt werden kann.

**Beispiel 3: Dichter Zellrasen aus Epithelzellen**

Epithele spielen in allen höher entwickelten Lebewesen eine sehr wichtige Rolle: Sie gewährleisten die Trennung von körperinneren und körperäußeren Bereichen und kontrollieren den Stofftransport (Wasser, Ionen, Proteine etc) in und aus dem Körper hinaus. Epithele sind meist einlagige Schichten spezialisierter Zellen, die zu einer durchgehenden (konfluenten) Schicht zusammenwachsen und zwischen den einzelnen Zellen spezialisierte Kontakte ausbilden, die so genannten "tight junctions". Diese Kontakte zeichnen sich dadurch aus, dass sich die Membranen der benachbarten Zellen sehr nahe kommen und so eine Barriere zwischen dem Raum oberhalb und unterhalb der Zellen bilden.

Abbildung 4 zeigt den Aufbau schematisch. Die durch die tight junctions hervorgerufene Impedanz trägt wesentlich zur Abdichtimpedanz bei. Bei einer gesunden und stabilen Zellschicht kann man davon ausgehen, dass die Adhäsion zum Substrat relativ konstant bleibt, so dass Variationen in der Abdichtimpedanz hauptsächlich den tight junctions zugeordnet werden können. Die Regulation der tight junctions läßt sich so in Echtzeit beobachten.

Durch die Epithelzellen kann zwischen Ober- und Unterseite ein Konzentrationsgradient aufgebaut werden, d.h. im Spalt zwischen Zellschicht und Substrat erfolgt eine An- oder Abreicherung bestimmter Stoffe, insbesondere Ionen, wodurch der Spalt $r_s$ und damit die Abdichtimpedanz beeinflusst wird. Wegen der hohen zeitlichen Auflösung des Verfahrens könnte auch ein durch die Zellen bewirkter Aufbau eines Konzentrationsgradienten beobachtet werden.

**Beispiel 4: Lipidmembran mit eingelagerten Transmembranproteinen**

Bei einer Lipiddoppelmembran, auch als 'supported lipid bilayer' bezeichnet, wird die Adhäsionsimpedanz aus dem Spalt $r_s$ sowie aus der Kapazität $c_m$ und der Leckleitfähigkeit $g_l$ der Lipidmembran gebildet (Abbildung 5). Eine Lipidmembran stellt ein einfaches Modellsystem für die Membranen biologischer Zellen dar. Insbesondere kann man Membranproteine und Ionenkanäle einlagern und deren Reaktion auf Botenstoffen untersuchen, wenn dadurch die Leckleitfähigkeit der Membran beeinflusst wird.

Mit Ionenkanälen lassen sich so gezielt Biosensoren bauen: Durch Verwendung ligandengesteuerter Ionenkanäle würde der Ligand die Öffnungswahrscheinlichkeit der Kanäle und damit die Abdichtimpedanz beeinflussen. Da eine große Zahl ligandengesteuerter Ionenkanäle existiert und durch gentechnische Methoden weitere Arten hergestellt werden können, lässt sich damit eine große Zahl spezifischer Biosensoren realisieren.

**Patentansprüche**

1. Verfahren zur Bestimmung der Abdichtimpedanz eines mit einer elektrisch leitfähigen Flüssigkeit gefüllten Spaltes zwischen einer Substanz und einem Träger, umfassend die Schritte

(a) Bereitstellen der Substanz auf dem Träger umfassend mindestens eine erste Elektrode in Kontakt mit der elektrisch leitfähigen Flüssigkeit, wobei die Substanz die mindestens eine erste Elektrode ganz oder teilweise bedeckt und wobei zwischen dem Träger und der Substanz durch Anlagerung der mit der elektrisch leitfähigen Flüssigkeit gefüllte Spalt entsteht,
(b) Bestimmen der Rauschspannung der Impedanz zwischen der mindestens einen ersten Elektrode und einer zweiten Elektrode, wobei die zweite Elektrode in Kontakt mit der elektrisch leitfähigen Flüssigkeit steht, und

(c) Bestimmen der Abdichtimpedanz aus der Rauschspannung.

2. Verfahren nach Anspruch 1, weiterhin umfassend den Schritt (d) Bestimmen der Adhäsion der Substanz auf dem Träger aus der Abdichtimpedanz, wobei eine hohe Abdichtimpedanz eine hohe Adhäsion charakterisiert.

3. Verfahren nach einem der Ansprüche 1 oder 2, wobei der Träger eine Mehrzahl von ersten Elektroden umfasst.

4. Verfahren nach einem der Ansprüche 1 bis 3, wobei die Bestimmung der Rauschspannung in Schritt (b) ohne Anlegen einer Spannung und eines Stroms zwischen der mindestens einen ersten und der zweiten Elektrode erfolgt.

5. Verfahren nach einem der vorstehenden Ansprüche, wobei in Schritt (c) die Abdichtimpedanz der Substanz auf dem Träger aus der Peak-Peak-Amplitude der Rauschspannung in einem vorbestimmten Frequenzbereich $f_1$ bis $f_2$ bestimmt wird, wobei die Peak-Peak-Amplitude derjenige Bereich ist, in welchem sich die Rauschspannung für bis zu 95% eines vorbestimmten Zeitraums bewegt.

6. Verfahren nach Anspruch 5, wobei die Peak-Peak-Amplitude derjenige Bereich ist, in welchem sich die Rauschspannung für bis zu 99,9% eines vorbestimmten Zeitraums bewegt.

7. Verfahren nach einem der Ansprüche, wobei in Schritt (c) die Abdichtimpedanz der Substanz auf dem Träger aus der Amplitude der Rauschspannung in einem vorbestimmten Frequenzbereich $f_1$ bis $f_2$ bestimmt wird, wobei die Amplitude der Rauschspannung durch $\pm 3{,}2\sigma$ gekennzeichnet ist, wobei $\sigma$ die Standardabweichung der Verteilung der einzelnen über eine vorbestimmte Zeit gemessenen Werte der Rauschspannung ist.

8. Verfahren nach einem der vorstehenden Ansprüche, wobei Schritt (c) umfasst:

   (c1) Bestimmen der Impedanz aus der Rauschspannung, und
   (c2) Bestimmen der Abdichtimpedanz aus der Impedanz.

9. Verfahren nach Anspruch 8, wobei in Schritt (c1) die Impedanz aus der zeitabhängigen Rauschspannung bestimmt wird.

10. Verfahren nach einem der vorstehenden Ansprüche, wobei Schritt (c) umfasst:

    (c1) Bestimmen der spektralen Leistungsdichte aus der Rauschspannung, und
    (c2) Bestimmen der Abdichtimpedanz der Substanz auf dem Träger aus der spektralen Leistungsdichte.

11. Verfahren nach Anspruch 10, wobei in Schritt (c1) die spektrale Leistungsdichte aus der zeitabhängigen Rauschspannung bestimmt wird.

12. Verfahren nach einem der Ansprüche 10 oder 11, wobei in Schritt (c2) die Abdichtimpedanz aus dem Integral der spektralen Leistungsdichte über einen vorbestimmten Frequenzbereich $f_1$ bis $f_2$ oder/und aus der Wurzel des Integrals der spektralen Leistungsdichte über einen vorbestimmten Frequenzbereich $f_1$ bis $f_2$ bestimmt wird.

13. Verfahren nach einem der vorhergehenden Ansprüche, wobei die Rauschspannung im Frequenzbereich von etwa 100 Hz bis etwa 100 kHz oder in einem Teilbereich hiervon bestimmt wird.

14. Verfahren nach einem der vorhergehenden Ansprüche, wobei die Schritte (b) und (c) mit zeitlichem Abstand zueinander ausgeführt werden.

15. Verfahren nach einem der Ansprüche 1 bis 14, wobei die Substanz eine biologische Substanz, insbesondere eine Zelle, ein Zellverband, eine Zellkultur oder eine Membran ist.

16. Verfahren nach Anspruch 15, wobei der Zellverband oder die Zellkultur eine Ein-Zell-Schicht, insbesondere eine konfluente Ein-Zell-Schicht, oder eine Mehr-Zell-Schicht bildet.

17. Verfahren nach einem der Ansprüche 1 bis 15, wobei die Substanz eine Lipidschicht ist, die gegebenenfalls Proteine enthält.

**18.** Verfahren nach einem der vorhergehenden Ansprüche, wobei die mindestens eine erste Elektrode ein Transistor, insbesondere ein Feldeffekttransistor, oder eine Metallelektrode ist.

**19.** Verfahren nach Anspruch 18, wobei die mindestens eine erste Elektrode eine Fläche des Trägers von höchstens 10 000 $\mu m^2$ bedeckt.

**20.** Verfahren nach einem der vorhergehenden Ansprüche, wobei die zweite Elektrode eine Ag/AgCl-Elektrode oder eine Elektrode wie in Anspruch 18 oder 19 definiert ist.

**21.** Verfahren nach einem der vorhergehenden Ansprüche, wobei die elektrisch leitfähige Flüssigkeit eine Salzlösung oder ein Zellkulturmedium ist.

**22.** Verfahren nach einem der vorhergehenden Ansprüche, wobei der Träger aus Silizium, Glas, einem Polymer, GaAs und GaN, Saphir oder/und Verbundwerkstoffen aus diesen Materialien gebildet wird.

**23.** Verfahren nach Anspruch 15 oder 16, wobei die Zelle, die Zellkultur oder der Zellverband auf dem Träger kultiviert wird.

**24.** Vorrichtung zur Durchführung des Verfahrens nach einem der vorhergehenden Ansprüche, umfassend

(i) einen Träger zur Aufnahme einer Substanz, umfassend mindestens eine erste Elektrode in Kontakt mit einer elektrisch leitfähigen Flüssigkeit,
(ii) Mittel zur Messung der Rauschspannung der Impedanz zwischen der mindestens einen ersten Elektrode und einer zweiten Elektrode, wobei die zweite Elektrode in Kontakt mit der elektrisch leitfähigen Flüssigkeit von (i) steht, und
(iii) Mittel zur Bestimmung der Abdichtimpedanz aus der Rauschspannung.

**25.** Vorrichtung nach Anspruch 24, weiterhin umfassend
(iv) Mittel zum Bestimmen der Adhäsion der Substanz auf dem Träger aus der Abdichtimpedanz der Substanz auf dem Träger.

**26.** Vorrichtung nach Anspruch 24 oder 25, worin der Träger eine Mehrzahl von ersten Elektroden umfasst.

**27.** Vorrichtung nach einem der Ansprüche 24 bis 26, worin die Mittel (ii) und (iii) voneinander getrennt sind.

**28.** Verfahren zur Identifizierung eines Wirkstoffs, der die Abdichtung einer Substanz auf einem Träger moduliert, umfassend die Schritte:

(I) Bestimmen der Abdichtimpedanz der Substanz auf einem Träger mit dem Verfahren nach einem der Ansprüche 1 und 3 bis 23 in Abwesenheit des zu untersuchenden Wirkstoffs,
(II) Bestimmen der Abdichtimpedanz der Substanz auf einem Träger mit dem Verfahren nach einem der Ansprüche 1 und 3 bis 23 in Anwesenheit des zu untersuchenden Wirkstoffs und
(III) Auswählen eines Wirkstoffs, der die Abdichtimpedanz der Substanz auf dem Träger auf vorbestimmte Weise moduliert.

**29.** Verfahren zur Identifizierung eines Wirkstoffs, der die Adhäsion einer Substanz auf einem Träger moduliert, umfassend die Schritte:

(1) Bestimmen der Adhäsion der Substanz auf einem Träger mit dem Verfahren nach Anspruch 2 und einem der Ansprüche 3 bis 23 in Abwesenheit des zu untersuchenden Wirkstoffs,
(2) Bestimmen der Adhäsion der Substanz auf einem Träger mit dem Verfahren nach Anspruch 2 und einem der Ansprüche 3 bis 23 in Anwesenheit des zu untersuchenden Wirkstoffs und
(3) Auswählen eines Wirkstoffs, der die Adhäsion der Substanz auf dem Träger auf vorbestimmte Weise moduliert.

**Claims**

1.  A method for determining the sealing impedance of a gap, filled with an electrically conductive liquid, between a substance and a support, comprising the steps

    (a) providing the substance on a support comprising at least one first electrode in contact with the electrically conductive liquid, wherein the substance entirely or partially covers the at least one first electrode and wherein the gap filled with the electrically conductive liquid is produced between the support and the substance by accumulation,

    (b) determining the noise voltage of the impedance between the at least one first electrode and a second electrode, wherein the second electrode is in contact with the electrically conductive liquid, and

    (c) determining the sealing impedance from the electrical noise.

2.  The method according to claim 1, furthermore comprising the step (d) determining the adhesion of the substance on the support from the sealing impedance, wherein a high sealing impedance characterises high adhesion.

3.  The method according to one of claims 1 or 2, wherein the support comprises a plurality of first electrodes.

4.  The method according to one of claims 1 to 3, wherein the noise in step (b) is determined without applying a voltage and a current between the at least one first and the second electrode.

5.  The method according to one of the preceding claims, wherein the sealing impedance of the substance on the support is determined in step (c) from the peak-to-peak amplitude of the noise voltage in a predetermined frequency range $f_1$ to $f_2$, wherein the peak-to-peak amplitude is the range in which the noise voltage moves for up to 95% of a predetermined time period.

6.  The method according to claim 5, wherein the peak-to-peak amplitude is the range in which the noise voltage moves for up to 99.9% of a predetermined time period.

7.  The method according to one of the claims, wherein the sealing impedance of the substance on the support is determined in step (c) from the amplitude of the noise voltage in a predetermined frequency range $f_1$ to $f_2$, wherein the amplitude of the noise voltage is **characterised by** $\pm 3.2\,\sigma$, wherein $\sigma$ is the standard deviation of the distribution of the individual values of the noise voltage measured over a predetermined time.

8.  The method according to one of the preceding claims, wherein step (c) comprises:

    (c1) determining the impedance from the noise voltage, and

    (c2) determining the sealing impedance from the impedance.

9.  The method according to claim 8, wherein the impedance is determined in step (c1) from the time-dependent noise voltage.

10. The method according to one of the preceding claims, wherein step (c) comprises:

    (c1) determining the spectral power density from the noise voltage, and

    (c2) determining the sealing impedance of the substance on the support from the spectral power density.

11. The method according to claim 10, wherein the spectral power density is determined in step (c1) from the time-dependent noise voltage.

12. The method according to one of claims 10 or 11, wherein the sealing impedance is determined in step (c2) from the integral of the spectral power density over a predetermined frequency range $f_1$ to $f_2$ or/and from the square root of the integral of the spectral power density over a predetermined frequency range $f_1$ to $f_2$.

13. The method according to one of the preceding claims, wherein the noise voltage is determined in a frequency range from approximately 100 Hz to approximately 100 kHz, or in a subregion thereof.

14. The method according to one of the preceding claims, wherein steps (b) and (c) are carried out with a time interval therebetween.

15. The method according to one of claims 1 to 14, wherein the substance is a biological substance, in particular a cell, a cell group, a cell culture or a membrane.

16. The method according to claim 15, wherein the cell group or the cell culture forms a single-cell layer, in particular a confluent single-cell layer, or a multiple-cell layer.

17. The method according to one of claims 1 to 15, wherein the substance is a lipid layer which may contain proteins.

18. The method according to one of the preceding claims, wherein the at least one first electrode is a transistor, in particular a field-effect transistor, or a metal electrode.

19. The method according to claim 18, wherein the at least one first electrode covers an area of the support of at most 10 000 $\mu m^2$.

20. The method according to one of the preceding claims, wherein the second electrode is an Ag/AgCl electrode, or is an electrode as defined in claim 18 or 19.

21. The method according to one of the preceding claims, wherein the electrically conductive liquid is a saline solution or a cell culture medium.

22. The method according to one of the preceding claims, wherein the support is formed from silicon, glass, a polymer, GaAs and GaN, sapphire or/and composite materials formed from these materials.

23. The method according to claim 15 or 16, wherein the cell, the cell culture or the cell group is cultivated on the support.

24. A device for carrying out the method according to one of the preceding claims, comprising

(i) a support for receiving a substance, comprising at least one first electrode in contact with an electrically conductive liquid,
(ii) means for measuring the noise voltage of the impedance between the at least one first electrode and a second electrode, wherein the second electrode is in contact with the electrically conductive liquid of (i), and
(iii) means for determining the sealing impedance from the noise voltage.

25. The device according to claim 24, furthermore comprising
(iv) means for determining the adhesion of the substance on the support from the sealing impedance of the substance on the support.

26. The device according to claim 24 or 25, wherein the support comprises a plurality of first electrodes.

27. The device according to one of claims 24 to 26, wherein the means (ii) and (iii) are separate from one another.

28. A method for identifying an active ingredient which modulates the sealing of a substance on a support, comprising the steps:

(I) determining the sealing impedance of the substance on a support with the method according to one of claims 1 and 3 to 23 in the absence of the active ingredient to be examined,
(II) determining the sealing impedance of the substance on a support with the method according to one of claims 1 and 3 to 23 in the presence of the active ingredient to be examined, and
(III) selecting an active ingredient which modulates the sealing impedance of the substance on the support in a predetermined manner.

29. A method for identifying an active ingredient which modulates the adhesion of a substance on a support, comprising the steps:

(1) determining the adhesion of the substance on a support with the method according to claim 2 and one of claims 3 to 23 in the absence of the active ingredient to be examined,

(2) determining the adhesion of the substance on a support with the method according to claim 2 and one of claims 3 to 23 in the presence of the active ingredient to be examined, and
(3) selecting an active ingredient which modulates the adhesion of the substance on the support in a predetermined manner.

**Revendications**

1. Procédé pour déterminer l'impédance de jonction d'un intervalle, rempli d'un liquide électriquement conducteur, entre une substance et un support, comprenant les étapes suivantes :

    (a) prévoir la substance sur le support comprenant au moins une première électrode en contact avec le liquide électriquement conducteur, dans lequel la substance couvre entièrement ou partiellement la au moins une première électrode, et dans lequel l'intervalle rempli du liquide électriquement conducteur se forme par fixation entre le support et la substance,
    (b) déterminer la tension de bruit de l'impédance entre la au moins une première électrode et une deuxième électrode, dans lequel la deuxième électrode est en contact avec le liquide électriquement conducteur, et
    (c) déterminer l'impédance de jonction à partir d'une tension de bruit.

2. Procédé selon la revendication 1, comprenant également
    (d) la détermination de l'adhésion de la substance sur le support à partir de l'impédance de jonction, dans lequel une impédance de jonction élevée caractérise une adhésion élevée.

3. Procédé selon l'une des revendications 1 à 2, dans lequel le support comprend une multiplicité de premières électrodes.

4. Procédé selon l'une des revendications 1 à 3, dans lequel la détermination de la tension de bruit lors de l'étape (b) a lieu sans application d'une tension et d'un courant entre la au moins une première électrode et la deuxième électrode.

5. Procédé selon l'une des revendications précédentes, dans lequel lors de l'étape (c), l'impédance de jonction de la substance sur le support est déterminée à partir de l'amplitude crête à crête de la tension de bruit dans une plage de fréquence $f_1$ à $f_2$ prédéfinie, dans lequel l'amplitude crête à crête est la zone dans laquelle la tension de bruit se déplace pour jusqu'à 95 % d'un laps de temps prédéfini.

6. Procédé selon la revendication 5, dans lequel l'amplitude crête à crête est la zone dans laquelle la tension de bruit se déplace pour jusqu'à 99,9 % d'un laps de temps prédéfini.

7. Procédé selon l'une des revendications, dans lequel lors de l'étape (c), l'impédance de jonction de la substance sur le support est déterminée à partir de l'amplitude de la tension de bruit dans une plage de fréquence $f_1$ à $f_2$ prédéfinie, dans lequel l'amplitude de la tension de bruit est **caractérisée par** $\pm$ 3,2 $\sigma$, $\sigma$ étant l'écart type de la distribution des valeurs individuelles de la tension de bruit mesurées sur une période prédéfinie.

8. Procédé selon l'une des revendications précédentes, dans lequel l'étape (c) comprend :

    (c1) une détermination de l'impédance à partir de la tension de bruit, et
    (c2) une détermination de l'impédance de jonction à partir de l'impédance.

9. Procédé selon la revendication 8, dans lequel lors de l'étape (c1), l'impédance est déterminée à partir de la tension de bruit dépendante du temps.

10. Procédé selon l'une des revendications précédentes, dans lequel l'étape (c) comprend :

    (c1) une détermination de la densité spectrale de puissance à partir de la tension de bruit,
    (c2) une détermination de l'impédance de jonction de la substance sur le support à partir de la densité spectrale de puissance.

11. Procédé selon la revendication 10, dans lequel lors de l'étape (c1), la densité spectrale de puissance est déterminée à partir de la tension de bruit dépendante du temps.

12. Procédé selon l'une des revendications 10 à 11, dans lequel lors de l'étape (c2), l'impédance de jonction est déterminée à partir de l'intégrale de la densité spectrale de puissance sur une plage de fréquence $f_1$ à $f_2$ prédéfinie ou/et de la racine de l'intégrale de la densité spectrale de puissance sur une plage de fréquence $f_1$ à $f_2$ prédéfinie.

13. Procédé selon l'une des revendications précédentes, dans lequel la tension de bruit est déterminée dans la plage de fréquence d'environ 100 Hz à environ 100 kHz ou dans une plage partielle de celle-ci.

14. Procédé selon l'une des revendications précédentes, dans lequel les étapes (b) et (c) sont réalisées de manière espacée dans le temps.

15. Procédé selon l'une des revendications 1 à 14, dans lequel la substance est une substance biologique, en particulier une cellule, un système cellulaire, une culture cellulaire ou une membrane.

16. Procédé selon la revendication 15, dans lequel le système cellulaire ou la culture cellulaire forme une couche unicellulaire, en particulier une couche unicellulaire confluente, ou une couche pluricellulaire.

17. Procédé selon l'une des revendications 1 à 15, dans lequel la substance est une couche lipidique qui contient éventuellement des protéines.

18. Procédé selon l'une des revendications précédentes, dans lequel la au moins une première électrode est un transistor, en particulier un transistor à effet de champ, ou une électrode métallique.

19. Procédé selon la revendication 18, dans lequel la au moins une première électrode couvre une surface du support de 10 000 $\mu$m$^2$ au maximum.

20. Procédé selon l'une des revendications précédentes, dans lequel la deuxième électrode est une électrode Ag/AgCl ou une électrode telle que déterminée dans la revendication 18 ou 19.

21. Procédé selon l'une des revendications précédentes, dans lequel le liquide électriquement conducteur est une solution saline ou un milieu de culture cellulaire.

22. Procédé selon l'une des revendications précédentes, dans lequel le support est formé de silicium, de verre, d'un polymère, de GaAs et GaN, de saphir ou/et de matériaux composites de ces matériaux.

23. Procédé selon la revendication 15 ou 16, dans lequel la cellule, la culture cellulaire ou le système cellulaire est cultivé sur le support.

24. Dispositif pour la mise en œuvre du procédé selon l'une des revendications précédentes, comprenant

(i) un support pour recevoir une substance, comprenant au moins une première électrode en contact avec un liquide électriquement conducteur,
(ii) des moyens pour mesurer la tension de bruit de l'impédance entre la au moins une première électrode et une deuxième électrode, dans lequel la deuxième électrode est en contact avec le liquide électriquement conducteur de (i), et
(iii) des moyens pour déterminer l'impédance de jonction à partir de la tension de bruit.

25. Dispositif selon la revendication 24, comprenant également (iv) des moyens pour déterminer l'adhésion de la substance sur le support à partir de l'impédance de jonction de la substance sur le support.

26. Dispositif selon la revendication 24 ou 25, dans lequel le support comprend une multiplicité de premières électrodes.

27. Dispositif selon l'une des revendications 24 à 26, dans lequel les moyens (ii) et (iii) sont séparés les uns des autres.

28. Procédé pour identifier une substance active qui module la jonction d'une substance sur un support, comprenant les étapes suivantes :

(I) déterminer l'impédance de jonction de la substance sur un support avec le procédé selon l'une des revendications 1 et 3 à 23 en l'absence de la substance active à analyser,

(II) déterminer l'impédance de jonction de la substance sur un support avec le procédé selon l'une des revendications 1 et 3 à 23 en présence de la substance active à analyser,

(III) sélectionner une substance active qui module d'une manière prédéfinie l'impédance de jonction de la substance sur le support.

29. Procédé pour identifier une substance active qui module l'adhésion d'une substance sur un support, comprenant les étapes suivantes :

(1) déterminer l'adhésion de la substance sur un support avec le procédé selon la revendication 2 et l'une des revendications 3 à 23 en l'absence de la substance active à analyser,

(2) déterminer l'adhésion de la substance sur un support avec le procédé selon la revendication 2 et l'une des revendications 3 à 23 en présence de la substance active à analyser, et

(3) sélectionner une substance active qui module d'une manière prédéfinie l'adhésion de la substance sur le support.

**Abbildung 1**

Badelektrode

Badwiderstand

Elektrolyt

isolierendes Objekt

Spalt

Meßelektrode
(z.B. FET, Metallelektrode)

Abdichtwiderstand

Spannungsverstärker bzw.
-meßinstrument

Substrat

**Abbildung 2**

isolierendes Objekt

Substrat

**Abbildung 3**

**Abbildung 4**

**Abbildung 5**

## Abbildung 6

## Abbildung 7

von den Transistoren 73 bis 80
gemessene Spannungen ($V_g$)

73
74
75
76
77
78
79
80

$\mathsf{I}$400 $\mu$V

200 ms

20 $\mu$m

Gatefläche des Transistors 81
(spannungssensitive Fläche) Transistoren

**Abbildung 8**

## Abbildung 9

von den Transistoren 73 bis 80
gemessene Spannungen ($V_0$)

73
|400 µV
74
75
76
77
78
79
80

60 ms  Zeitdauer des
Kontakts

20 µm

Transistoren

## Abbildung 10

**Abbildung 11**

**Abbildung 12**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4920047 A
- US 6611151 B
- US 5888374 A
- US 2003113833 A1

**In der Beschreibung aufgeführte Nicht-Patentliteratur**

- **GIAEVER ; KEESE.** ECIS. *Nature,* 1993, vol. 366, 591-592
- **VASILESCU et al.** *Electrochimica Acta,* 1974, vol. 19, 181-186
- **M. YANG et al.** Influence of geometry and environmental parameters on the quality of signature patterns for single neuron chemical sensors. *SENSORS AND ACTUATORS B104,* 03. Januar 2005, 163-171
- **J. WEGENER et al.** Barrier function of porcine choroid plexus epithelial cells is modulated by cAMP-dependent pathways in vitro. *rain Research,* 2000, vol. 853, 115-124
- **GIAEVER I. et al.** A morphological biosensor for mammalian cells. *Nature,* 09. Dezember 1993, vol. 366
- **BUITENWEG J.R. et al.** Measurement of sealing resistance of cell-electrode interfaces in neuronal cultures using impedance spectroscopy. *Medical and Biological Engineering and Computing,* 01. September 1998, vol. 36 (5), 630-637
- **HILLE, B.** Ionic Channels of Excitable Membranes. Sinauer Associates Inc, 1991